# EUROPEAN PATENT SPECIFICATION

(11) **EP 0 460 217 B1**
(45) Date of publication and mention of the grant of the patent: **09.11.1994**
(21) Application number: 90917547.3
(22) Date of filing: 28.11.1990
(51) Int. Cl.: C12N 15/40, C12N 15/82, C07K 13/00, C12Q 1/68

(54) **cDNA OF ORSV GENE**
CDNS DES ORSV-VIRUS GENS
ADNc DU GENE DU VIRUS ORSV

(30) Priority: 28.11.1989 JP 306626/89
(43) Date of publication of application: 11.12.1991
(73) Proprietor: Nippon Oil Co., Ltd., Tokyo 105 (JP)
(72) Inventor: ISOMURA, Keisuke, Kawasaki-shi Kanagawa 211 (JP); MATSUMOTO, Yoshinori, Yamaguchi 740 (JP); CHATANI, Masaaki, Yamaguchi 744 (JP); IKEGAMI, Masato, Tokyo 194-02 (JP)
(74) Representative: Ritter, Stephen David
(86) International application number: JP9001546
(87) International publication number: WO9108296

(56) References cited:
- EP-A- 0 223 452
- VAN REGENMORTEL 'The Plant Viruses Volume 2' 1986 , PLENUM PRESS , NEW YORK
- PROC. NATL ACAD. SCI. USA vol. 79, 1982, pages 5818 - 5822 P. GOELET ET AL. 'Nucleotide sequence of tobacco mosaic virus RNA'
- THE EMBO JOURNAL vol. 6, no. 2, 1987, pages 307 - 311 N. TAKAMATSU ET AL. 'Expression of bacterial CAT gene in tobacco plants mediated by TMV-rna'
- PROC. NATL. ACAD. SCI. USA. vol. 83, 1986, pages 5043 - 5047 T. MESHI ET AL. 'In vitro transcription of infectious RNAs from full-length cDNAs of tobacco mosaic virus'
- Berichte des Ohara Instituts für Landwirtschaftliche Biologie Bd XIII; Heft 3, pp. 149-159, August 1966.
- Nogaka Kenkya, vol 60, no 2, pp. 53-67, 1983

## Description

### FIELD OF THE INVENTION

The present invention relates to DNA corresponding to an Odontoglossum ringspot virus (ORSV) gene, and a fragment or complementary strand thereof.

More particularly, the present invention relates to isolated or cloned, cyclic or linear DNA corresponding to the Odontoglossum ringspot virus gene, and the fragment or complementary strand thereof.

The DNA, fragment and complementary strand of Odontoglossum ringspot virus gene according to the present invention are useful as starting materials in the construction of valuable vectors for plants. They are also useful for preparing virus-resistant orchids.

Further, the DNA, fragment and complementary strand of Odontoglossum ringspot virus gene according to the present invention are useful as probes for screening virus genes.

Still, the DNA, fragment and complementary strand of Odontoglossum ringspot virus gene according to the present invention can be ligated with a suitable expression vector to produce a virus-related protein or peptide by the expression in a host cell. Such produced virus-related proteins or peptides are also useful as starting materials in the manufacture of reagents for screening virus genes.

### BACKGROUND OF THE INVENTION

Odontoglossum ringspot virus (ORSV) is a member of cylindrical RNA viruses which are contagious mainly among orchids such as Cattleya, Cymbidium, Dendrobium, Odontoglossum, etc. Virus particles themselves have been isolated (Nugaku Kenkyu, Vol. 60, Nov. 2, 53-67, 1983); however, the gene thereof itself has not yet been obtained. Thus, no information relating to the genomic structure as well as the base sequence of the gene has yet been disclosed. Heretofore, it is impossible to utilize the gene of Odontoglossum ringspot virus.

In general, genomic DNA derived from viruses is widely developed and employed as a vector among not only animal cells but also other host cells in recombinant DNA techniques. In case of plants, however, it is technically difficult to use plant virus-derived genomic RNA in DNA recombination because most of plant viruses are constituted by RNA genes. Therefore, it is necessary to prepare complementary DNA (cDNA) by using a template of viral RNA.

For example, Ti plasmid which is carried in Agrobacterium tumefaciens capable of forming tumors in Dicotyledoneae plants such tomato and tabacco and the DNA derived from cauliflower mosaic virus which produces a disease into cabbage, Brassica campestris, etc. are heretofore found as potential vectors for DNA recombination of plant genes. Almost all of plant viruses are classified into a group of RNA viruses. Moreover, their propagation activities are extremely high. From these observations, it can be assumed that plant cells may maintain some physiological conditions suitable for replication of RNA.

In view of the circumstance, it is expected that RNA-vector systems utilizing those derived from RNA viruses would be extremely useful in DNA manipulation for plant genes. However, there is heretofore no valuable vector in the prior art except for Ti plasmid which is incorporated into a chromosome of plant cell wherein an amount of the expression is quite low.

It is also expected that RNA-vectors constructed by utilizing RNA viruses would express more than Ti plasmid because of the chromosome-independent auto-synthesis.

From the above-mentioned reasons, the incorporation of foreign genes with Ti plasmid has focused merely on plant breeding and the main topic has been directed to the induction of herbicide-resistance genes. Still the introduction of new auto-replicable RNA vectors for plants will develop and restore the advantage of plant cell or body in production of valuable proteins such as animal hormones.

Moreover, the production of valuable proteins in E. coli has faced a large number of difficulties upon practical uses. One of them is that in case of increased expression the expressed foreign proteins are frequently packaged as inclusion bodies. Since E. coli is an autotrophic single cell, the excess production of foreign proteins has a bad effect on the micro-organism. Therefore, the formation of inclusion bodies is purposeful. This is an unavoidable drawback in the production of valuable proteins in E. coli host.

On the other hand, plant bodies are very tolerant. For example, even if one of leaves dies, it affects little all plant bodies. From characteristics of plant, strong exclusion mechanisms including denaturation and insolubilization hardly occur even in an excess production of foreign proteins.

Accordingly, it is valuable to develop as a RNA vector cDNA corresponding to the Odontoglossum ringspot virus gene. Since the cDNA contains genes for viral coat proteins, it is expected that the cDNA may be ligated with Ti plasmid to produce virus-resistant plants by incorporating the resultant vector into a chromosome of plant cell.

PNAS, USA, vol. 79, pages 5818-5822 1982 describes the nucleotide sequence of TMV and EP-A-0223452 discloses expression of TMV proteins in bacteria.

The Plant Viruses, volume 2, pages 233-247 1986 describes the properties of ORSV, but provides no information on its genetic make-up. In comparing ORSV with other viruses, it is stated that some of the so-called isolates of "TMV-O" could be considered as synonymous with ORSV but that ORSV should be considered as a virus distinct from other tobamoviruses such as U2-TMV, tomato mosaic virus and TMV.

The present inventors have succeeded in isolating an Odontoglossum ringspot virus RNA gene, preparing a cDNA thereof by utilizing the isolated virus RNA gene as a template, cloning the cDNA, and expressing the viral gene on the cloned cDNA in a host cell. The present inventors have found that the cDNA possesses suitable restriction sites.

The present inventors have also succeeded in determining the base sequence of the cDNA corresponding to the Odontoglossum ringspot virus gene. As a result, the present inventors have found that novel vectors and transformants can be produced by DNA recombination based on such disclosed base sequence and the virus may be detected through utilization of the isolated cDNA.

### DISCLOSURE OF THE INVENTION

The present invention relates to DNA corresponding to Odontoglossum ringspot virus (ORSV) genes, specifically those isolated from orchids such as Cattleya, Cymbidium, Dendrobium, Odontoglossum, etc., and a fragment or complementary strand thereof.

The Odontoglossum ringspot viruses intended in the present invention are not limited to those isolated from orchids but include those having the characteristics identical with the virus as mentioned hereinbelow.

The Odontoglossum ringspot viruses intended in the present invention can be mutated naturally or artificially by the treatment with mutagens such as nitrosourea, ultra-violet and radiation without serious damage to their contagiousness.

A part of Odontoglossum ringspot virus gene may be modified by substitutions, deletions, or additions on the basis of the information on the disclosed base sequence thereof.

It may be supplemented with genes derived from other viruses, micro-organisms, animals and plants.

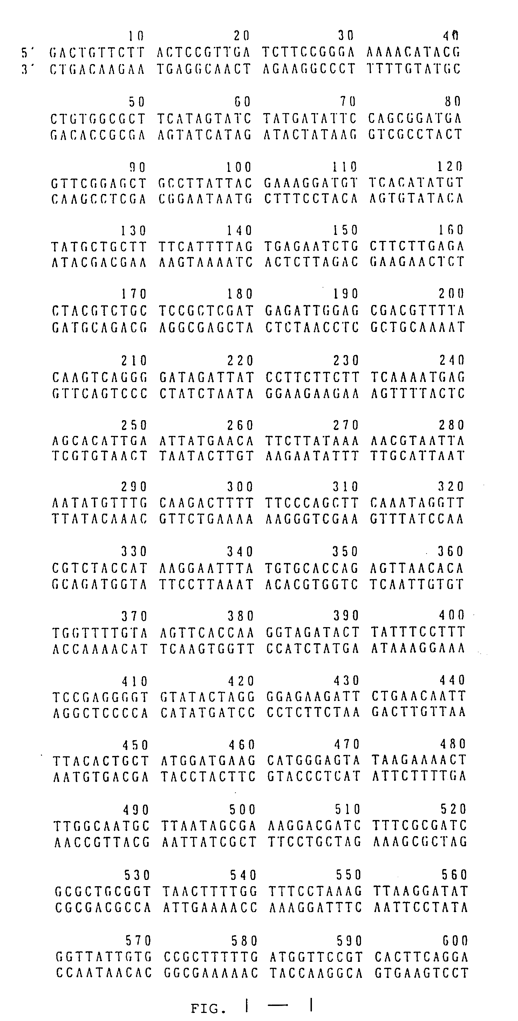

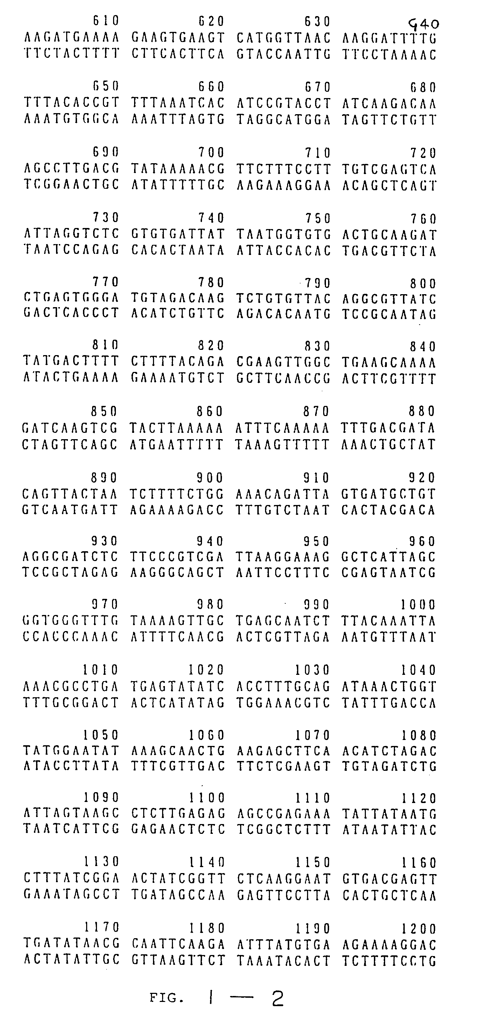

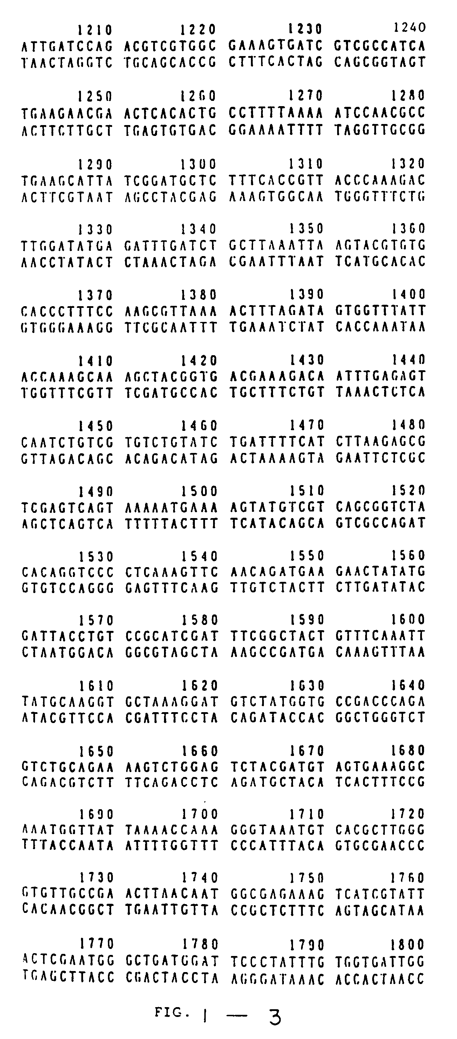

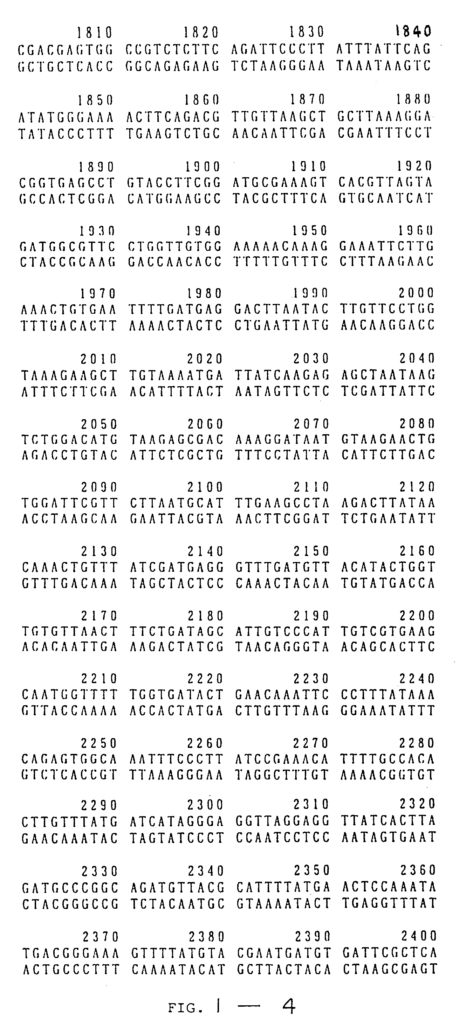

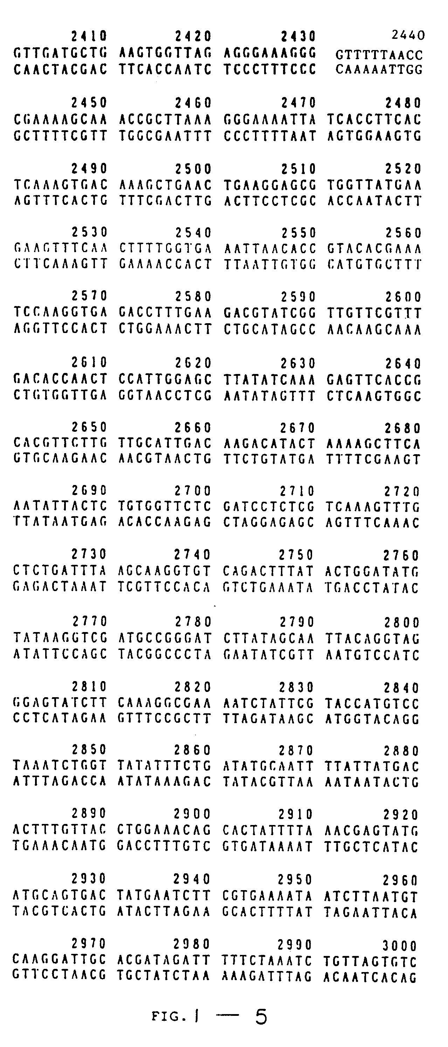

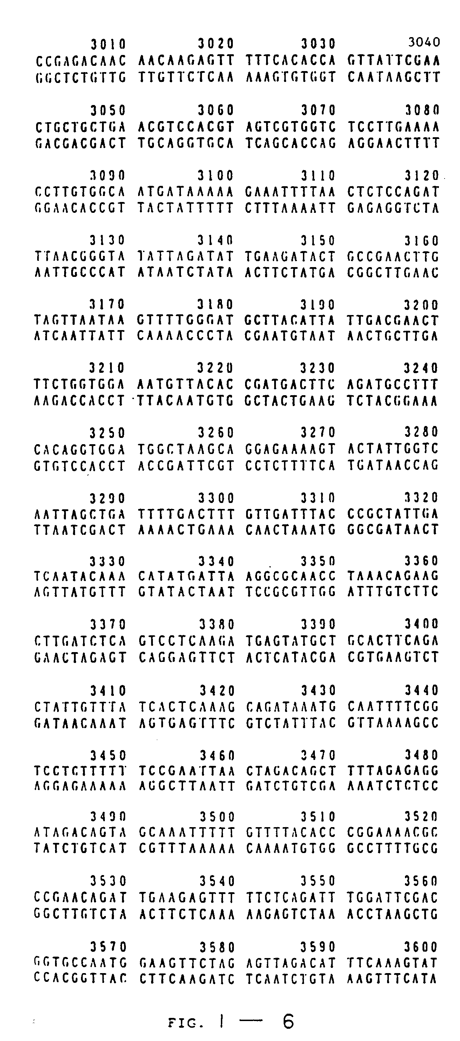

The DNA corresponding to Odontoglossum ringspot virus gene according to the present invention means an optional fragment or full-length of double stranded DNA comprising the base sequence as illustrated in Figure 1. In Figure 1, the upper base sequence represents a cDNA chain corresponding to (+) RNA strand of Odontoglossum ringspot virus genome. Such DNA includes those coding for either 33 K protein or coat protein or both, of Odontoglossum ringspot virus gene.

The present invention also relates to not only the DNA strand complementary to viral RNA but another strand.

The present invention still relates to an optional DNA fragment of the base sequence as illustrated in Figure 1. Such fragments can be derived from the deoxynucleotide base sequence as illustrated in Figure 1 by a suitable known restriction enzyme alone or in a combination with a plurality thereof.

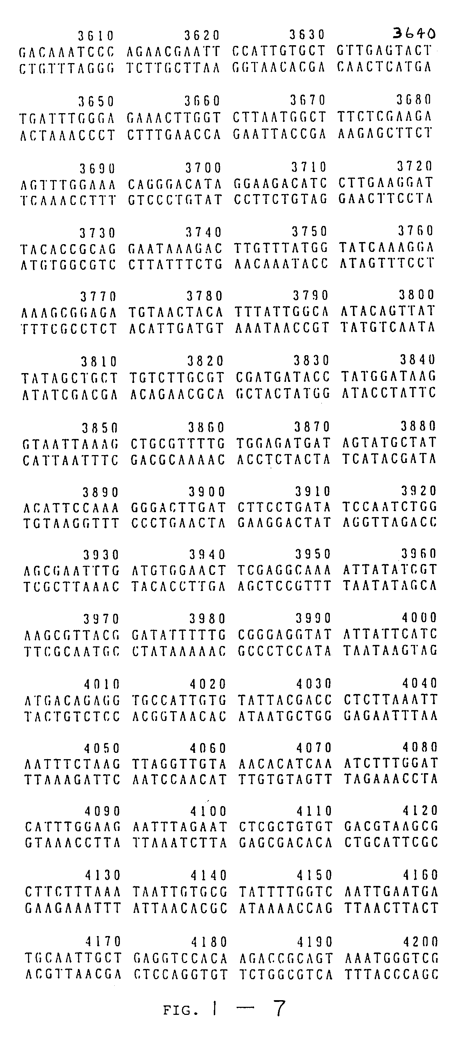

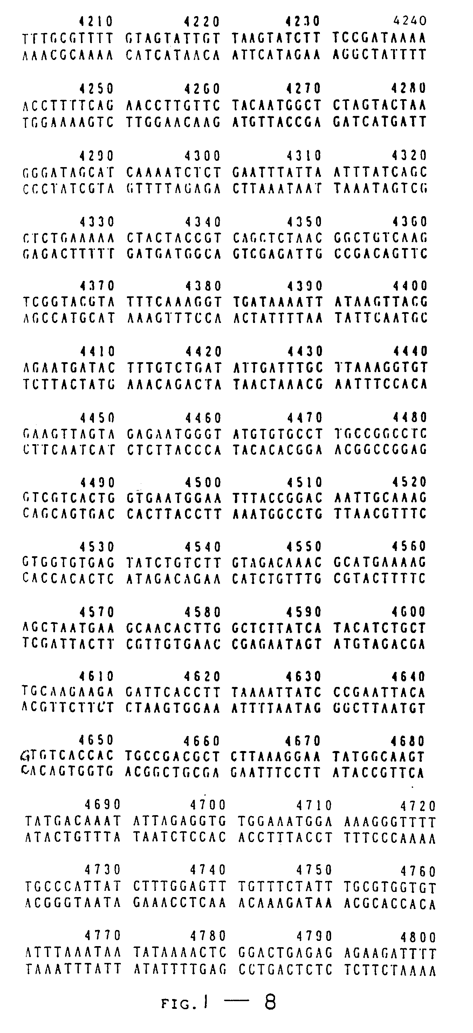

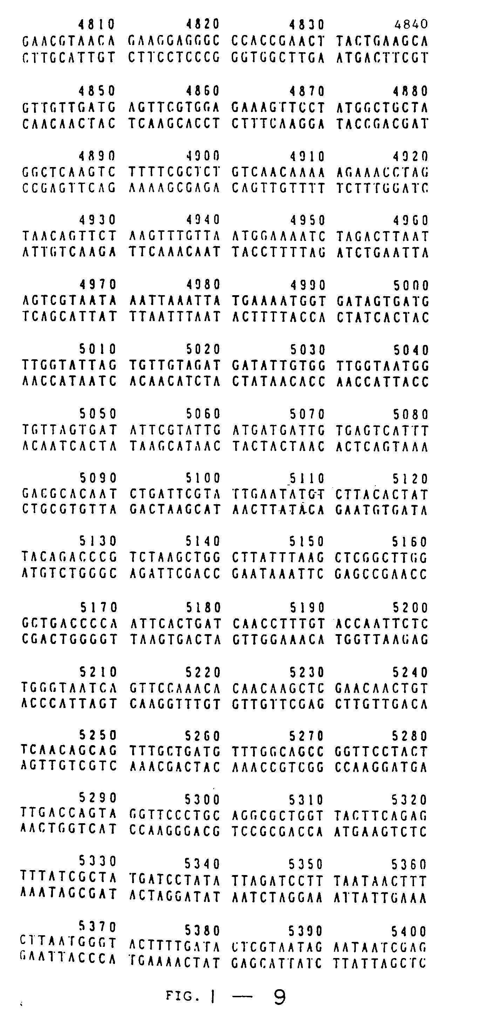

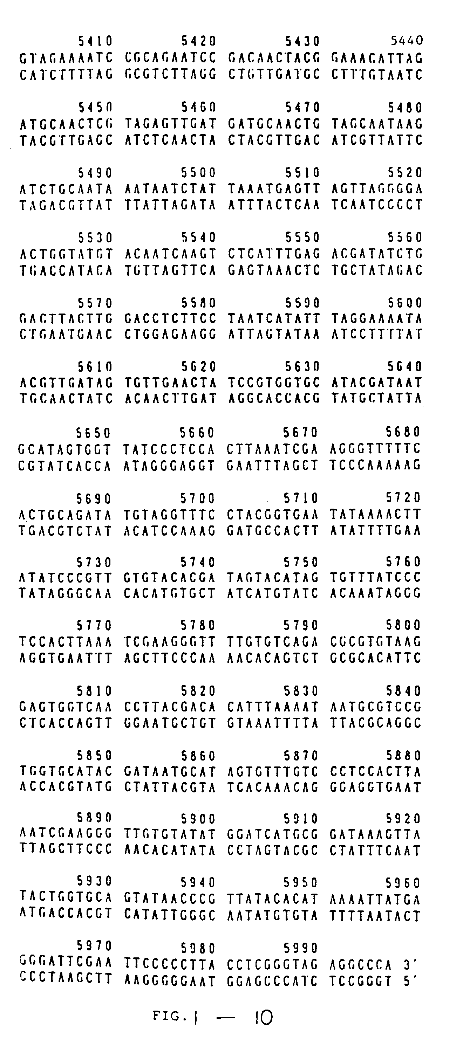

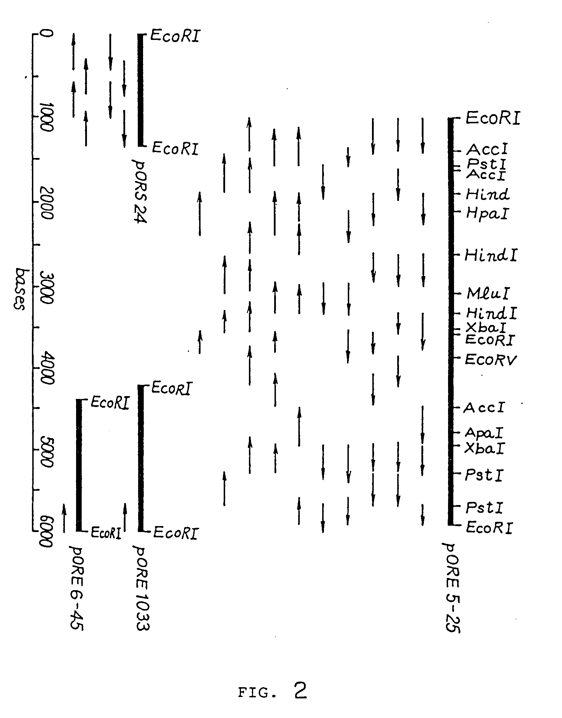

Examples of such restriction enzymes include those as described, for example, in Protein, Nucleic Acid and Enzyme, Vol. 30, No. 13, p.1429-1445 (1985). Examples of such DNA fragments include about 3.87 Kbp, about 1.58 Kbp, and about 1.58 Kbp fragments which are derived from DNA comprising the base sequence as illustrated in Figure 1, which is cleaved at two sites by the treatment with Cla I. Further examples of the DNA fragment include those derived by the treatments with each restriction enzyme as depicted in Figure 2.

It is clear that the base sequences of the DNA fragments which are obtained by digesting the DNA of Figure 1 with said restriction enzyme can be determined by a recognition specificity each of suitable restriction enzymes and the deoxynucleotide base sequence as illustrated in Figure 1.

The cDNA according to the present invention may be modified. Such modification may include cleavages, deletions, insertions, additions, substitutions and ligations of DNA chains.

The DNA thus obtained according to the present invention can be either digested with exonuclease or ligated with an oligonucleotide or polynucleotide.

The present invention still relates to DNA capable of hybridizing with any of an optional fragment and entire sequence of the bases as illustrated in Figure 1.

Examples of such DNA include those which have essentially the functions either identical with or similar to any of portions and entire genes in the Odontoglossum ringspot virus and may be cDNA corresponding to the gene, and a fragment or complementary strand thereof.

The DNA corresponding to the Odontoglossum ringspot virus according to the present invention can be prepared by processes including isolation of virus particles from plants infected with the subject virus, separation of viral RNA from the virus particles, and synthesis by reverse transcriptase in the use of viral RNA as a template. The DNA thus obtained is ligated with a suitable vector for cloning, incorporated into a suitable host cell, and cloned. The DNA thus cloned can be cleaved with a suitable restriction enzyme and sequenced by Maxam-Gilbert method, the chain termination method, etc.

The DNA sequence thus disclosed can be employed on the basis of the information. For example, the DNA sequence can be employed as a probe for examining gene libraries derived from naturally occurring genes or by the shot gun method to separate RNA and/or its corresponding cDNA which has substantially the same function as in Odontoglossum ringspot virus.

The present invention further relates to an assay method for detecting Odontoglossum ringspot virus genes, their corresponding cDNA and nucleic acid fragments.

Still the present invention relates to a method for cloning DNA corresponding to the detected Odontoglossum ringspot virus gene, and a fragment or complementary strand thereof and their products.

The present invention relates to a recombinant vector which carries DNA corresponding to the Odontoglossum ringspot virus gene, and a fragment or complementary strand thereof.

More specifically, the present invention relates to a recombinant vector which carries DNA containing any of optional fragment and entire sequence of double-stranded DNA with the base sequence as depicted in Figure 1, a fragment or complementary strand thereof.

The recombinant vector may be hybrid DNA which is prepared by inserting the DNA corresponding to the Odontoglossum ringspot virus gene, fragment or complementary strand thereof according to the present invention into a vector known, widely utilizable, or commercially available in the art, or suitably prepared from another vector alone or in combination thereof.

Examples of such genes include E. coli plasmid vectors, cosmid vectors, phage vectors, plasmid vectors for Bacillus, plasmid vectors derived from Staphylococcus, vectors for yeast, shuttle vectors self-replicable in plural host cells, etc. These vectors are usually carrying a suitable promoter, restriction sites, and markers for selection; however, it is preferable to add a necessary function to the vector depending on necessity.

Examples of such E. coli plasmid vectors include pBR322, pBR325, pBR328, pUC19, pUC118, pUC119, pMB9, pKC7, etc. Examples of such cosmid vectors include pKY2662, pHC79, pJC720, etc. Examples of such phage vectors include λgt11, λgt · λC, λgt · λB', λ1059, EMBL3.4, λWES · λB', Charon 4A, Charon 28, λ_{L} 47-1, Charon 8, Charon 17, Charon 20, Charon 3A, Charon 16A, Charon 13A, Charon 14A, Charon 17A, etc. Examples of such plasmid vectors for Bacillus include pUB110, pUB112, pHY300PLK, pTA1060, pTA1015, pTA1020, pTA1050, pTP4, pTP5, etc. Examples of such plasmid vectors derived from Staphylococcus include pSA0501, pC194, pC221, pC223, pUB110, pUB112, etc. Examples of such vectors for yeast include YRp-type vectors, YEp-type vectors, YRp vectors, YEp-type vectors, YRMp-type vectors such as YRp7, Y1p32, YEp13, pYC1, pYC2, YIp1, pJDB248, pJDB219, pJDB248, pDB248, pGK-1,2, pLC544, pFYM2, and pFYM225. Examples of such shuttle vectors include pFYM225, YRp7, Y1p5-derived plasmids, pTA1302, and those replicable in plural host cells. The shuttle vector may include those wherein a suitable promoter or regulating sequence is incorporated and those derived by the combination of vectors each other.

The hybrid vectors can be prepared by cleaving the double-stranded DNA containing the base sequence as illustrated in Figure 1, with a restriction enzyme capable of cutting at a suitable site or plural sites, and ligating the resulting fragment to a cleaved vector which is produced by the same restriction enzyme species.

The hybrid vectors of the present invention can be prepared as follows:
RNA extracted from the subject virus is employed as a template for synthesizing cDNA, which is then treated with a DNA ligase such as T4 DNA ligase to bind EcoRI adaptors to both sides of cDNA fragment. The resulting DNA is mixed with the product which is produced from the above-mentioned vector, for example, pUC118 by the degradation with EcoRI followed by the treatment with alkaline phosphatase. The resulting mixture is treated with a DNA ligase such as T4 DNA ligase to bind and cyclize.

The alkaline phosphatase treatment can be substituted with other dephosphorylation. Such phosphatase treatment is preferable for preventing self-cyclization.

Examples of the restriction enzymes used in these processes include EcoRI, AccI, PstI, HindIII, MluI, XbaI, EcoRV, ApaI, etc. The restriction enzyme may include those as described, for example, in Protein, Nucleic Acid and Enzyme, Vol. 30, No. 13, p.1429-1445 (1985) or other known enzymes.

The hybrid DNA thus obtained can be introduced into a suitable host cell. The hybrid DNA from E. coli plasmid vectors, plasmid vectors for Bacillus, Staphylococcus-derived plasmid vectors, vectors for yeast, and shuttle vectors can be introduced into E. coli, Bacillus, Staphylococcus, yeast, and suitable host cells according to conventional methods, respectively.

In case of E. coli and Bacillus, the suitable host cell is first converted through CaCl₂ treatment into a competent cell which is subsequently transformed with the hybrid DNA thus obtained according to conventional methods.

In case of yeast, the host cell is treated with cell wall lytic enzyme such as glusulase, helicase, and zymolase, and a formed protoplast is then transformed, for example, in the presence of PEG and Ca ⁺⁺ or by treatments with an alkaline metal ion such Li⁺, or a reducing reagent such as 2-mercaptoethanol.

The transformant thus obtained can be selected through drug-resistance or nutrient demand, or colony hybridization.

The hybrid DNA may contain other foreign genes by the insertion. Such foreign genes may obtained from viruses, plants, animals, bacteria, fungi, etc. or synthesized chemically.

The foreign gene used may preferably include those utilizable in transforming plants. Examples of such foreign genes include resistance-determinants against pests or parasites, resistance-determinants against pathogens, factors relating to resistance or tolelance against herbicides, growth-regulators, those for producing industrially useful or valuable proteins or peptides, or other substances, and the like.

Examples of such resistance-determinants against pests or parasites include insecticidal toxin producing genes against insects or nematodes, genes for inducing protenase-inhibitors, genes for producing ribosome-inactivating proteins, etc.

Examples of such factors relating to resistance or tolelance against herbicides include paraquat resistance-determinants, sulfuron resistance-determinants, sulfonamide resistance-determinants, glyphosate resistance-determinants, etc.

Examples of such resistance-determinants against pathogens include virus resistance-determinants, other resistance-determinants against various bacterial toxins, resistance factors relating to kanamycin, etc.

Examples of such growth-regulators include genes for giving improved resistance against high or low temperature, deficiency of water, fertlizers or nutrients, etc. The growth-regulators also include those for controlling naturally occurring enzymes, stored proteins, etc. in plants, industrially useful or valuable proteins or peptides, or other substances, and the like.

The foreign genes may include other genes which are useful or valuable when they are introduced into plants.

In a preferred embodiment, the transformation according to the present invention is further illustrated hereinbelow.

EcoRI adaptors are ligated into both sides of DNA as illustrated in Figure 1 with T4 DNA ligase and the product is then ligated into EcoRI fragment of E. coli plasmid vector, pUC118 with T4 DNA ligase to form hybrid DNA. E. coli JM109 is transformed with the hybrid DNA and the resulting transformant is incubated in Agar medium (100 mM IPTG and 2% gal on the surface of 2×TY) containing 50 µg/ml of ampicillin. The colonies are applied to the blue-white selection method (selection by expression of lac-Z gene) and the white colonies are selected.

The colonies are transformed E. coli JM109 which carries the hybrid DNA wherein the DNA sequence of Figure 1 is inserted at the EcoRI site of plasmid, pUC118. The hybrid DNA can be isolated from the transformant by known methods.

A base sequence of cDNA corresponding to a full-length of Odontoglossum ringspot virus gene can be determined step by step by repeating the above-mentioned procedure.

The hybrid DNA may be inserted into the vector as mentioned hereinbelow, without or after cleavage. Alternatively, the hybrid DNA may be treated suitably to form a new hybrid DNA and the resultant hybrid DNA may be reintroduced into a host cell. The transformant thus obtained may also be propagated.

Mass production of the subject hybrid DNA can be achieved by multiplying the hybrid DNA-containing transformant, for example, E. coli JM109, applying the treatment for increasing only the hybrid DNA depending on necessity, lysing the cells and isolating the hybrid DNA from an aqueous solution containing the same through ultracentrifugation, electrophoresis, etc.

In case of the hybrid DNA derived from E. coli plasmid, pUC118, the hybrid DNA-containing E. coli transformant is applied to the multiplication of hybrid DNA according to known methods, and then lysed. The resulting DNA solution is applied to cesium chloride density gradient ultracentrifugation to afford covalently closed circular hybrid DNA.

The isolated hybrid DNA is treated with the same restriction enzyme as for cloning so that DNA corresponding to Odontoglossum ringspot virus gene, or fragment thereof (more specifically, an optional part or full-length of the base sequence as illustrated in Figure 1) can be separated from the vector DNA fragment.

The DNA fragment thus obtained is applied to gel electrophoresis employing agalose gel or polyacrylamide to separate each other. The gel carrying a band of the desired DNA fragment is cut out and the purified DNA fragment is extracted and isolated from the separated gel according to known methods.

The DNA fragment thus obtained can be inserted into the expression hybrid DNA and the resulting recombinant DNA can be introduced into a suitable host cell for expression.

According to the present invention, 33K proteins and coat proteins of Odontoglossum ringspot virus can be obtained by expression in the host cell.

The proteins according to the present invention may be modified by DNA recombination. Such modification may include cleavages, deletions, insertions, additions, substitutions and ligations.

The 33K proteins and coat proteins of Odontoglossum ringspot virus as well as peptide fragments thereof include any of those modified products.

The above-mentioned 33K protein of Odontoglossum ringspot virus has functions analogous to those of tobacco mosaic virus (TMV) 33K protein.

The recombinant vectors of the present invention may include those hybrid DNAs which are prepared by inserting the DNA corresponding to Odontoglossum ringspot virus (ORSV) gene, fragment or complementary strand thereof, into a vector capable of transforming plant cells or tissues. The vectors capable of transforming plant cells or tissues are those widely known, frequently utilizable, or commercially available in the art.

Examples of such vectors capable of transforming plant cells or tissues may include Ti plasmids or derivatives thereof, Ri plasmids or derivatives thereof, califlower mosaic virus derived vectors, geminivirus derived vectors, shuttle vectors recognized as vectors for yeast, etc. Ti plasmid and Ri plasmid are carried in the gram-negative bacterial family of Rhizobiaceae comprising Agrobacteria such as Agrobacterium tumefaciens and Agrobacterium rhizogenes. Such Ti plasmid or Ri plasmid is inducing crown gall disease or rhizogenic disease. The vectors capable of transforming plant cells or tissues also include those capable of autonomously replicating in plant cells or transforming plant cells.

Examples of the Ti plasmids or derivatives thereof include those classified by each opine, e.g. octopine, nopaline, agropine, those having T-DNA and those constructed by the combination of genes relating to other opine degradations, oncogenes, agrosin sensitivity, as well as genes relating to replication.

Examples of such plasmids include pMON-type vectors, disarm-type plasmids such as pGV3850, binary vectors, etc.

Representative examples of such plasmids include pMON200, pMON505, pMON237, ect. as described in S. G. Rogers, et al., Recombinant DNA methodology, Ed by Ray Wu, et al., Academic Press, p387-410 (1989).

The hybrid DNA for plants is useful in transforming plants. When Ti plasmid or derivative thereof is employed, the transformation may include introduction of the hybrid DNA into Agrobacterium tumefaciens by direct transfer of autonomously movable vectors through hybridization between three or two parents, direct incorporation, spheroplast fusion with other cells containing the hybrid DNA, liposome methods, or transfection after packaging, followed by infection with Agrobacterium tumefaciens. In case of Ri plasmid or derivative thereof, Agrobacterium rhizogenes is employed in the same manner as for Agrobacterium tumefaciens.

The methods widely used by the skilled persons can be employed in infecting plants with Agrobacteria. Such methods may include addition of Agrobacteria to cultured plant cells or tissues followed by incubation, direct infection to cultivated plants or protoplasts, and cell fusion between plant protoplasts and bacterial spheroplasts.

The hybrid DNA of the present invention is useful in transforming plant cells or tissues by direct introduction. Such processes include, for example, incorporation of plant protoplasts together with carrier DNA into plant cells in the presence of polyethylene glycol, etc., insertion of the hybrid DNA into liposome composed of phosphatidyl serine and cholesterol followed by the treatment of plant protoplasts with a high pH solution containing a high concentration of calcium in the presence of polyethylene glycol, micro-injection and electoporation.

The plant cells or tissues thus transformed can be regenarated by conventional methods to form plants. Such plants can be cultivated even in fields or greenhouses.

The present invention relates to the transformants which are produced by transforming host cells with the recombinant vector which carries DNA corresponding to the Odontoglossum ringspot virus gene, and fragment or complementary strand thereof.

The present invention relates to the methods for producing the transformants.

The present invention also relates to a method for the hybridization using any of DNA corresponding to the Odontoglossum ringspot virus gene, fragment and complementary strand thereof.

More specifically, the present invention relates to a method for the hybridization using any of DNA strands containing an optional fragment or entire sequence of double-stranded DNA with the base sequence as depicted in Figure 1.

The hybridization is carried out according to various known techniques. Examples of such hybridization are illustrated hereinbelow.

Viral DNA is fixed on nitrocellulose filters, and the filter is hybridized in a buffer, for example, the buffer containing 25 mM KPO₄ (pH 7.4), 5×SSC, 5×Deuhardt solution (containing 0.2% phycol (molecular weight: 40,000), 0.2% polyvinylpyrrolidone (molecular weight: 30,000-40,000), and 0.2% bovine serum albumin (fraction V)), 50 µg/ml salmon sperm DNA, and 50% formaldehyde, at 37°C-42°C for 3 hours to overnight.

The nitrocellulose filter thus treated is hybridized in a buffer containing DNA probe composed of a segment of the corresponding viral DNA according to the present invention, for example, together with 25 mM KPO₄ (pH 7.4), 5 ×SSC, 5×Deuhardt solution, 50 µg/ml salmon sperm DNA, 50% formaldehyde, and 10% dextran sulfate at 37°C-42°C for 6 hours to overnight, and assayed for hybridized viral DNA.

General and conventional methods for DNA recombination, which are employed in the present invention, are further described in T. Manniatis et al., Molecular Cloning - A Laboratory Manual, Cold Spring Habor Laboratory (1982); R. Wu, et al., Methods in Enzymology, 68 (1979), 100 (1983) and 101 (1983), Academic Press; etc.

### PREFERRED EMBODIMENT

The following examples are intended to illustrate the invention in further detail and should by no means be construed as limiting the scope of the invention.

### Example 1. Isolation of Virus RNA

Odontoglossum ringspot virus-infected Cymbidium leaves were freezed in liquid nitrogen, pulverized, suspended in phosphate buffer (pH 7.0) containing 0.1% thioglycol, and then filtered through gauze.

The suspension was then treated with carbon tetrachloride, and polyethylene glycol 6,000, NaCl and Triton X-100 (TM) were added to achieve 6% polyethylene glycol 6,000, 0.1M NaCl, and 1% Triton X-100, respectively. The resulting suspension was allowed to stand on ice for 2 hours. Then the solution was centrifuged (3,000 rpm, 15 min, 4 °C, TOMY-RS 20HB, Japan) to obtain a fraction containing virus particles. To the fraction was added phosphate buffer and the suspension was formed. The suspension was centrifuged (30,000 rpm, 1 hour, 4°C, BECKMAN L8-70M 70.1 Ti rotor, U.S.A) to obtain virus particles as a precipitate.

The resulting virus particles were then applied to centrifugation (25,700 rpm, 2 hours, 4°C, BECKMAN SW 40 Ti, U.S.A.) in 10%-40% sucrose density gradient.

The virus was purified by fractionation through the bottom of the centrifuge tube.

The virus RNA was extracted from the purified virus particles by the SDS-phenol method according to conventional methods.

### Example 2. Preparation of cDNA from Virus RNA

Poly(A) was ligated to the 3' terminus of purified virus RNA as obtained in Example 1, by treatment with poly (A) polymerase.

The first DNA strand complementary to virus RNA was produced by AMV reverse transcriptase and oligo d(T) as a primer according to a conventional method.

A synthetic primer which is prepared by referring to information of viral base sequence may be used instead of the above-mentioned oligo d(T) primer. The hybrid DNA consisting of viral RNA-DNA thus obtained was treated with RNase H so that the RNA chain was nicked. The second DNA strand complementary to the first DNA strand was synthesized by DNA polymerase I in the presence of said nicked RNA as a primer. Both ends of cDNA were treated with Klenow fragment to remove the overhanging ends and create blunt ends.

In order to practise the foregoing treatments, various kits and reagents attached thereto, commercially available from Amasham, Japan, Pharmacia, Sweden, Cosmo Bio, Japan, Seikagagaku Kougyo K.K., Daiichi Chemical Co. Ltd., Takara Shuzou K.K., Wako Pure Chemical K.K., etc. can be used.

### Example 3. Cloning of Viral cDNA

DNA prepared in Example 2 was treated with T4 DNA ligase according to a conventional method to attach EcoRI adaptors to both termini.

Although the DNA thus obtained can be cloned by insertion into various vectors according to a conventional method, this example illustrates herein cloning by use of Esherichia coli plasmid pUC118. The DNA wherein the EcoRI adaptor was added at both sides of DNA prepared in Example 2 as described above was treated with polynucleotide kinase according to a conventional method to phosphorylate the EcoRI adaptor portion thereof.

The plasmid pUC118 was cleaved with restriction enzyme EcoRI at 37 °C for 2 hours. The linear pUC118 thus obtained was treated with phenol to inactivate the restriction enzyme, and dephosphorylated with an alkaline phosphatase according to a conventional method.

Each DNA thus obtained was recovered by ethanol precipitation and dissolved in ligation buffer (66 mM Tris-HCl (pH 7.6), 6.6 mM MgCl₂, 10 mM DTT, and 0.1 mM ATP). Then these solution were mixed and T4 DNA ligase was added to the mixed solution followed by incubation at 16°C for 2 hours according to a conventional method to achieve ligation.

Transformation of competent E. coli JM109 with the reaction mixture was carried out by the calcium chloride method according to a conventional method. The transformed E. coli JM109 was selected by utilizing indications that it was ampicillin resistant and incapable of expressing a Lac-Z gene, thereby E. coli colonies were finally obtained which carry plasmids inserted with DNA or its fragment corresponding to the Odontoglossum ringspot virus gene into the EcoRI site of pUC118. Four colonies were selected from theE. coli colonies thus obtained. The plasmids which were inserted with DNA or its fragment corresponding to the Odontoglossum ringspot virus, were isolated from these colonies.

The plasmids thus obtained are designated as pORE5-25, pORE6-45, pORE1033 and pORS24. The region coded by each plasmid is schematically illustrated in Figure 2.

In order to practise the above procedures, various kits and reagents commercially available as mentioned in Example 2 can be used.

### Example 4.

### Determination of Viral cDNA Base Sequeuce

The base sequence of DNA was determined by employing the plasmid obtained in Example 3 according to sequencing strategies as illustrated in Figure 2.

The sequencing of its DNA was carried out by fragmentation with a suitable restriction enzyme, cloning of DNA fragments to be determined with M13 phage vector as developed by J. Messing et al. or pUC118/119 plasmid vector and then the dideoxy method as developed by F. Sauger et al.(Protein, Nucleic Acid and Enzyme, Vol. 29, No.4, p.294-306(1984)).

Each fragment is inserted into the cloning site of pUC118 or pUC119 in order to analyze base sequences starting from the site each of EcoRI, AccI, PstI, HindIII, MluI, XbaI, EcoRV and ApaI as shown in Figure 2; however, when it is impossible to fragmentate to a suitable size, the sequencing is achieved after it is treated with exonuclease III to create various fragments having an appropriate size. The DNA base sequence corresponding to the Odontoglossum ringspot virus gene as determined according to the above mentioned procedures is shown in Figure 1.

Although the DNA base sequence as illustrated in Figure 1 is not identical with a full length of odontoglossum ringspot virus gene, it contains regions encoding a 33K protein and a coat protein of Odontoglossum ringspot virus.

In the sequence illustrated in Figure 1, the upper sequence represents cDNA corresponding to the (+) chain of Odontoglossum ringspot virus genome.

When from the 5107th to the 5583th base region of the base sequence illustrated in Figure 1 was expressed, expression products having viral coat protein activity are obtained. In Figure 1, the upper sequence between two chains stands for a sequence having a direction from 5'-terminus to 3'-terminus and the lower sequence stands for a sequence complementary to the upper sequence and having a direction from left side 3'-terminus to 5'-terminus.

### Example 5. Expression of Virus Protein

The following examples illustrate that a gene coding for a viral protein is separated from the base sequence illustrated in Figure 1, a vector for expression is constructed from the gene, then a host cell is transformed with said vector, and the host cell is cultivated to produce a protein which is determined a virus protein.

### (1) Separation of Viral Coat protein Gene.

Open reading frames (ORF) were checked in the base sequence as illustrated in Figure 1.

Among the checked ORF, one ORF was selected which has a size and location, similar to and is capable of coding for a protein with a molecular weight similar to that of tabacco mosaic virus (TMV) coat protein by comparing a genomic structure of TMV. The 5107th to the 5583th base sequence as illustrated in Figure 1 is estimated as the gene coding for the Odontoglossum ringspot virus coat protein.

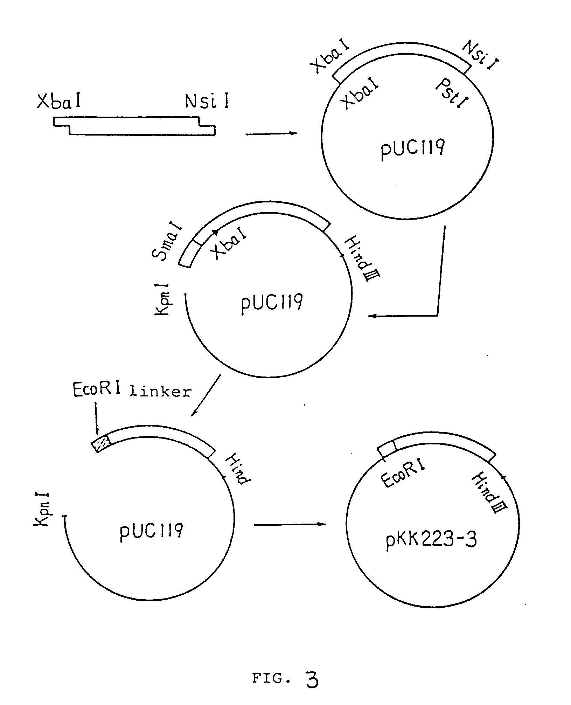

Then said DNA fragment was cut out by restriction enzyme. First DNA fragments carrying the gene portion which was estimated to encode the coat protein, for example, pORE5-25, pORE1033 and pORE6-45, were treated with the restriction enzymes XbaI (the 4950th) and NsiI (the 5639th) according to the conventional method as described in Example 3 to obtain a XbaI-NsiI fragment. A vector for expression was constructed from said XbaI-NsiI fragment as shown in Figure 3. This XbaI-NsiI fragment was ligated to the XbaI and PstI site of plasmid vector pUC119 according to the conventional method as described in Example 3. The hybrid DNA thus obtained was introduced into E. coli JM109 according to the conventional method as illustrated in Example 3 and subcloned. Then transformed E. coli colonies were selected.

The subject subcloned hybrid plasmid DNA was recovered from the transformed E. coli colonies. The plasmid was treated with restriction enzymes KpnI and SmaI according to the standard method as illustrated in Example to make it linear. The linear plasmid was deleted with exonuclease III according to a conventional method. This deletion was continued until only four bases remain prior to the initiation site, ATG for translating the gene ORF coding for the viral coat protein.

To the 5'-terminal blunt end was ligated an EcoRI linker and the plasmid was treated with HindIII according to a conventional method to obtain an EcoRI-HindIII fragment carrying the ORF gene for the viral coat protein.

### (2) Expression of Viral Coat Protein Gene

The DNA fragment carrying viral coat protein gene was ligated into the EcoRI HindIII site of the expression vector pKK223-3 for E. coli according to the standard method as described in Example 3. The competent E. coli JM109 was transformed with the hybrid DNA according to the standard method as described in Example 3.

The E.coli transformant thus obtained was incubated in 2×TY medium 5 ml) containing ampicillin (50 µg) overnight. An aliquot (50 µl) of the culture was further incubated in a fresh medium (2×TY medium, 5 ml)(37 °C, 2.5 hrs). To the culture was added 5 µl of 500 mM IPTG (final concentration: 0.1mM) followed by incubation at 40°C for 1.5 hours. The culture thus obtained was ice-cooled for 5 minutes. The E. coli was recovered from 2 ml of the culture according to a conventional method, washed with TE buffer and suspended in 100 µl of NaKPO₄ (pH 7.0). This suspention was disrupted by ultrasonication (one cycle including 1 min, sonication and 30 sec. internal, was repeated six times) to solubilize proteins from E. coli. The solubilized proteins were applied to an electrophoresis on 15% SDS polyarylamide gel and then proteins on the gel were electroblotted to nitrocellulose filters. The filters were examined for the presence of protein capable of reacting with anti-serum to odortoglussom ring spot virus by using an immunoblotting assay (Western Blotting Assay) according to a conventional method.

As shown in Figure 3, the protein extracted from the transformed E. coli JM109 (designated as pORCP-D83) was reacted with anti-serum to Odontoglussum ringspot virus. This isolated protein was identified with Odontoglussum ringspot viral coat protein.

The above-mentioned transformant E. coli JM109 (herein designated as pORCP-D83) was deposited with Fermentation Research Institute Agency of Industrial Science and Technology, MITI, Japan on November 27, 1989 and assigned as FERM P-11134.

### (3) By repeating the similar procedures, the 33K viral protein is obtained.

### BRIEF DESCRIPTION OF DRAWINGS

Fig. 1 illustrates a polynucleotide base sequence of Odontoglossum ringspot virus gene. The upper base sequence represents cDNA corresponding to a (+) strand of viral genome.

Fig. 2 depicts a strategy for DNA sequencing and main restriction sites employed.

Each arrow indicates a direction for sequencing the base together with a length of DNA fragments.

Fig. 3 illustrates a construction of expression vector for Odontoglossum ringspot virus coat protein.

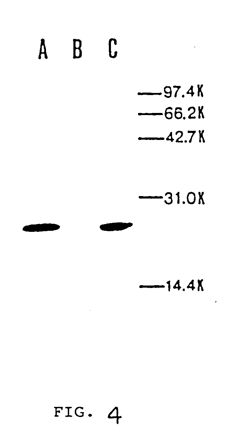

Fig. 4 shows the results of immunoblotting assay for identifying recombinant Odontoglossum rignspot virus coat proteins.

Lane A, Lane B and Lane C represent Odontoglossum ringspot virus particles, proteins extracted from E. coli and proteins extreated from transformant E. coli JM109 (pORCP-D83), respectively.

## Claims

1. An isolated DNA sequence corresponding to (1) an Odontoglossum ringspot virus (ORSV) genome, or a fragment thereof produced by cleavage with one or more restriction enzymes selected from EcoRI, AccI, PstI, Hind III, HpaI, MluI, XbaI and ApaI, or (2) a sequence complementary to (1).

2. A DNA sequence according to Claim 1 in which the ORSV genome has the sequence:

10 20 30 40
5′ GACTGTTCTT ACTCCGTTGA TCTTCCGGGA AAAACATACG
3′ CTGACAAGAA TGAGGCAACT AGAAGGCCCT TTTTGTATGC

50 60 70 80
CTGTGGCGCT TCATAGTATC TATGATATTC CAGCGGATGA
GACACCGCGA AGTATCATAG ATACTATAAG GTCGCCTACT

90 100 110 120
GTTCGGAGCT GCCTTATTAC GAAAGGATGT TCACATATGT
CAAGCCTCGA CGGAATAATG CTTTCCTACA AGTGTATACA

130 140 150 160
TATGCTGCTT TTCATTTTAG TGAGAATCTG CTTCTTGAGA
ATACGACGAA AAGTAAAATC ACTCTTAGAC GAAGAACTCT

170 180 190 200
CTACGTCTGC TCCGCTCGAT GAGATTGGAG CGACGTTTTA
GATGCAGACG AGGCGAGCTA CTCTAACCTC GCTGCAAAAT

210 220 230 240
CAAGTCAGGG GATAGATTAT CCTTCTTCTT TCAAAATGAG
GTTCAGTCCC CTATCTAATA GGAAGAAGAA AGTTTTACTC

250 260 270 280
AGCACATTGA ATTATGAACA TTCTTATAAA AACGTAATTA
TCGTGTAACT TAATACTTGT AAGAATATTT TTGCATTAAT

290 300 310 320
AATATGTTTG CAAGACTTTT TTCCCAGCTT CAAATAGGTT
TTATACAAAC GTTCTGAAAA AAGGGTCGAA GTTTATCCAA

330 340 350 360
CGTCTACCAT AAGGAATTTA TGTGCACCAG AGTTAACACA
GCAGATGGTA TTCCTTAAAT ACACGTGGTC TCAATTGTGT

370 380 390 400
TGGTTTTGTA AGTTCACCAA GGTAGATACT TATTTCCTTT
ACCAAAACAT TCAAGTGGTT CCATCTATGA ATAAAGGAAA

410 420 430 440
TCCGAGGGGT GTATACTAGG GGAGAAGATT CTGAACAATT
AGGCTCCCCA CATATGATCC CCTCTTCTAA GACTTGTTAA

450 460 470 480
TTACACTGCT ATGGATGAAG CATGGGAGTA TAAGAAAACT
AATGTGACGA TACCTACTTC GTACCCTCAT ATTCTTTTGA

490 500 510 520
TTGGCAATGC TTAATAGCGA AAGGACGATC TTTCGCGATC
AACCGTTACG AATTATCGCT TTCCTGCTAG AAAGCGCTAG

530 540 550 560
GCGCTGCGGT TAACTTTTGG TTTCCTAAAG TTAAGGATAT
CGCGACGCCA ATTGAAAACC AAAGGATTTC AATTCCTATA

570 580 590 600
GGTTATTGTG CCGCTTTTTG ATGGTTCCGT CACTTCAGGA
CCAATAACAC GGCGAAAAAC TACCAAGGCA GTGAAGTCCT

610 620 630 640
AAGATGAAAA GAAGTGAAGT CATGGTTAAC AAGGATTTTG
TTCTACTTTT CTTCACTTCA GTACCAATTG TTCCTAAAAC

650 660 670 680
TTTACACCGT TTTAAATCAC ATCCGTACCT ATCAAGACAA
AAATGTGGCA AAATTTAGTG TAGGCATGGA TAGTTCTGTT

690 700 710 720
AGCCTTGACG TATAAAAACG TTCTTTCCTT TGTCGAGTCA
TCGGAACTGC ATATTTTTGC AAGAAAGGAA ACAGCTCAGT

730 740 750 760
ATTAGGTCTC GTGTGATTAT TAATGGTGTG ACTGCAAGAT
TAATCCAGAG CACACTAATA ATTACCACAC TGACGTTCTA

770 780 790 800
CTGAGTGGGA TGTAGACAAG TCTGTGTTAC AGGCGTTATC
GACTCACCCT ACATCTGTTC AGACACAATG TCCGCAATAG

810 820 830 840
TATGACTTTT CTTTTACAGA CGAAGTTGGC TGAAGCAAAA
ATACTGAAAA GAAAATGTCT GCTTCAACCG ACTTCGTTTT

850 860 870 880
GATCAAGTCG TACTTAAAAA ATTTCAAAAA TTTGACGATA
CTAGTTCAGC ATGAATTTTT TAAAGTTTTT AAACTGCTAT

890 900 910 920
CAGTTACTAA TCTTTTCTGG AAACAGATTA GTGATGCTGT
GTCAATGATT AGAAAAGACC TTTGTCTAAT CACTACGACA

930 940 950 960
AGGCGATCTC TTCCCGTCGA TTAAGGAAAG GCTCATTAGC
TCCGCTAGAG AAGGGCAGCT AATTCCTTTC CGAGTAATCG

970 980 990 1000
GGTGGGTTTG TAAAAGTTGC TGAGCAATCT TTACAAATTA
CCACCCAAAC ATTTTCAACG ACTCGTTAGA AATGTTTAAT

1010 1020 1030 1040
AAACGCCTGA TGAGTATATC ACCTTTGCAG ATAAACTGGT
TTTGCGGACT ACTCATATAG TGGAAACGTC TATTTGACCA

1050 1060 1070 1080
TATGGAATAT AAAGCAACTG AAGAGCTTCA ACATCTAGAC
ATACCTTATA TTTCGTTGAC TTCTCGAAGT TGTAGATCTG

1090 1100 1110 1120
ATTAGTAAGC CTCTTGAGAG AGCCGAGAAA TATTATAATG
TAATCATTCG GAGAACTCTC TCGGCTCTTT ATAATATTAC

1130 1140 1150 1160
CTTTATCGGA ACTATCGGTT CTCAAGGAAT GTGACGAGTT
GAAATAGCCT TGATAGCCAA GAGTTCCTTA CACTGCTCAA

1170 1180 1190 1200
TGATATAACG CAATTCAAGA ATTTATGTGA AGAAAAGGAC
ACTATATTGC GTTAAGTTCT TAAATACACT TCTTTTCCTG

1210 1220 1230 1240
ATTGATCCAG ACGTCGTGGC GAAAGTGATC GTCGCCATCA
TAACTAGGTC TGCAGCACCG CTTTCACTAG CAGCGGTAGT

1250 1260 1270 1280
TGAAGAACGA ACTCACACTG CCTTTTAAAA ATCCAACGCC
ACTTCTTGCT TGAGTGTGAC GGAAAATTTT TAGGTTGCGG

1290 1300 1310 1320
TGAAGCATTA TCGGATGCTC TTTCACCGTT ACCCAAAGAC
ACTTCGTAAT AGCCTACGAG AAAGTGGCAA TGGGTTTCTG

1330 1340 1350 1360
TTGGATATGA GATTTGATCT GCTTAAATTA AGTACGTGTG
AACCTATACT CTAAACTAGA CGAATTTAAT TCATGCACAC

1370 1380 1390 1400
CACCCTTTCC AAGCGTTAAA ACTTTAGATA GTGGTTTATT
GTGGGAAAGG TTCGCAATTT TGAAATCTAT CACCAAATAA

1410 1420 1430 1440
ACCAAAGCAA AGCTACGGTG ACGAAAGACA ATTTGAGAGT
TGGTTTCGTT TCGATGCCAC TGCTTTCTGT TAAACTCTCA

1450 1460 1470 1480
CAATCTGTCG TGTCTGTATC TGATTTTCAT CTTAAGAGCG
GTTAGACAGC ACAGACATAG ACTAAAAGTA GAATTCTCGC

1490 1500 1510 1520
TCGAGTCAGT AAAAATGAAA AGTATGTCGT CAGCGGTCTA
AGCTCAGTCA TTTTTACTTT TCATACAGCA GTCGCCAGAT

1530 1540 1550 1560
CACAGGTCCC CTCAAAGTTC AACAGATGAA GAACTATATG
GTGTCCAGGG GAGTTTCAAG TTGTCTACTT CTTGATATAC

1570 1580 1590 1600
GATTACCTGT CCGCATCGAT TTCGGCTACT GTTTCAAATT
CTAATGGACA GGCGTAGCTA AAGCCGATGA CAAAGTTTAA

1610 1620 1630 1640
TATGCAAGGT GCTAAAGGAT GTCTATGGTG CCGACCCAGA
ATACGTTCCA CGATTTCCTA CAGATACCAC GGCTGGGTCT

1650 1660 1670 1680
GTCTGCAGAA AAGTCTGGAG TCTACGATGT AGTGAAAGGC
CAGACGTCTT TTCAGACCTC AGATGCTACA TCACTTTCCG

1690 1700 1710 1720
AAATGGTTAT TAAAACCAAA GGGTAAATGT CACGCTTGGG
TTTACCAATA ATTTTGGTTT CCCATTTACA GTGCGAACCC

1730 1740 1750 1760
GTGTTGCCGA ACTTAACAAT GGCGAGAAAG TCATCGTATT
CACAACGGCT TGAATTGTTA CCGCTCTTTC AGTAGCATAA

1770 1780 1790 1800
ACTCGAATGG GCTGATGGAT TCCCTATTTG TGGTGATTGG
TGAGCTTACC CGACTACCTA AGGGATAAAC ACCACTAACC

1810          1820          1830          1840
CGACGAGTGG CCGTCTCTTC AGATTCCCTT ATTTATTCAG
GCTGCTCACC GGCAGAGAAG TCTAAGGGAA TAAATAAGTC

1850          1860          1870          1880
ATATGGGAAA ACTTCAGACG TTGTTAAGCT GCTTAAAGGA
TATACCCTTT TGAAGTCTGC AACAATTCGA CGAATTTCCT

1890          1900          1910          1920
CGGTGAGCCT GTACCTTCGG ATGCGAAAGT CACGTTAGTA
GCCACTCGGA CATGGAAGCC TACGCTTTCA GTGCAATCAT

1930          1940          1950          1960
GATGGCGTTC CTGGTTGTGG AAAAACAAAG GAAATTCTTG
CTACCGCAAG GACCAACACC TTTTTGTTTC CTTTAAGAAC

1970          1980          1990          2000
AAACTGTGAA TTTTGATGAG GACTTAATAC TTGTTCCTGG
TTTGACACTT AAAACTACTC CTGAATTATG AACAAGGACC

2010          2020          2030          2040
TAAAGAAGCT TGTAAAATGA TTATCAAGAG AGCTAATAAG
ATTTCTTCGA ACATTTTACT AATAGTTCTC TCGATTATTC

2050          2060          2070          2080
TCTGGACATG TAAGAGCGAC AAAGGATAAT GTAAGAACTG
AGACCTGTAC ATTCTCGCTG TTTCCTATTA CATTCTTGAC

2090          2100          2110          2120
TGGATTCGTT CTTAATGCAT TTGAAGCCTA AGACTTATAA
ACCTAAGCAA GAATTACGTA AACTTCGGAT TCTGAATATT

2130          2140          2150          2160
CAAACTGTTT ATCGATGAGG GTTTGATGTT ACATACTGGT
GTTTGACAAA TAGCTACTCC CAAACTACAA TGTATGACCA

2170          2180          2190          2200
TGTGTTAACT TTCTGATAGC ATTGTCCCAT TGTCGTGAAG
ACACAATTGA AAGACTATCG TAACAGGGTA ACAGCACTTC

2210          2220          2230          2240
CAATGGTTTT TGGTGATACT GAACAAATTC CCTTTATAAA
GTTACCAAAA ACCACTATGA CTTGTTTAAG GGAAATATTT

2250          2260          2270          2280
CAGAGTGGCA AATTTCCCTT ATCCGAAACA TTTTGCCACA
GTCTCACCGT TTAAAGGGAA TAGGCTTTGT AAAACGGTGT

2290 2300 2310 2320
CTTGTTTATG ATCATAGGGA GGTTAGGAGG TTATCACTTA
GAACAAATAC TAGTATCCCT CCAATCCTCC AATAGTGAAT

2330 2340 2350 2360
GATGCCCGGC AGATGTTACG CATTTTATGA ACTCCAAATA
CTACGGGCCG TCTACAATGC GTAAAATACT TGAGGTTTAT

2370 2380 2390 2400
TGACGGGAAA GTTTTATGTA CGAATGATGT GATTCGCTCA
ACTGCCCTTT CAAAATACAT GCTTACTACA CTAAGCGAGT

2410 2420 2430 2440
GTTGATGCTG AAGTGGTTAG AGGGAAAGGG GTTTTTAACC
CAACTACGAC TTCACCAATC TCCCTTTCCC CAAAAATTGG

2450 2460 2470 2480
CGAAAAGCAA ACCGCTTAAA GGGAAAATTA TCACCTTCAC
GCTTTTCGTT TGGCGAATTT CCCTTTTAAT AGTGGAAGTG

2490 2500 2510 2520
TCAAAGTGAC AAAGCTGAAC TGAAGGAGCG TGGTTATGAA
AGTTTCACTG TTTCGACTTG ACTTCCTCGC ACCAATACTT

2530 2540 2550 2560
GAAGTTTCAA CTTTTGGTGA AATTAACACC GTACACGAAA
CTTCAAAGTT GAAAACCACT TTAATTGTGG CATGTGCTTT

2570 2580 2590 2600
TCCAAGGTGA GACCTTTGAA GACGTATCGG TTGTTCGTTT
AGGTTCCACT CTGGAAACTT CTGCATAGCC AACAAGCAAA

2610 2620 2630 2640
GACACCAACT CCATTGGAGC TTATATCAAA GAGTTCACCG
CTGTGGTTGA GGTAACCTCG AATATAGTTT CTCAAGTGGC

2650 2660 2670 2680
CACGTTCTTG TTGCATTGAC AAGACATACT AAAAGCTTCA
GTGCAAGAAC AACGTAACTG TTCTGTATGA TTTTCGAAGT

2690 2700 2710 2720
AATATTACTC TGTGGTTCTC GATCCTCTCG TCAAAGTTTG
TTATAATGAG ACACCAAGAG CTAGGAGAGC AGTTTCAAAC

2730 2740 2750 2760
CTCTGATTTA AGCAAGGTGT CAGACTTTAT ACTGGATATG
GAGACTAAAT TCGTTCCACA GTCTGAAATA TGACCTATAC

2770 2780 2790 2800
TATAAGGTCG ATGCCGGGAT CTTATAGCAA TTACAGGTAG
ATATTCCAGC TACGGCCCTA GAATATCGTT AATGTCCATC

2810 2820 2830 2840
GGAGTATCTT CAAAGGCGAA AATCTATTCG TACCATGTCC
CCTCATAGAA GTTTCCGCTT TTAGATAAGC ATGGTACAGG

2850 2860 2870 2880
TAAATCTGGT TATATTTCTG ATATGCAATT TTATTATGAC
ATTTAGACCA ATATAAAGAC TATACGTTAA AATAATACTG

2890 2900 2910 2920
ACTTTGTTAC CTGGAAACAG CACTATTTTA AACGAGTATG
TGAAACAATG GACCTTTGTC GTGATAAAAT TTGCTCATAC

2930 2940 2950 2960
ATGCAGTGAC TATGAATCTT CGTGAAAATA ATCTTAATGT
TACGTCACTG ATACTTAGAA GCACTTTTAT TAGAATTACA

2970 2980 2990 3000
CAAGGATTGC ACGATAGATT TTTCTAAATC TGTTAGTGTC
GTTCCTAACG TGCTATCTAA AAAGATTTAG ACAATCACAG

3010 3020 3030 3040
CCGAGACAAC AACAAGAGTT TTTCACACCA GTTATTCGAA
GGCTCTGTTG TTGTTCTCAA AAAGTGTGGT CAATAAGCTT

3050 3060 3070 3080
CTGCTGCTGA ACGTCCACGT AGTCGTGGTC TCCTTGAAAA
GACGACGACT TGCAGGTGCA TCAGCACCAG AGGAACTTTT

3090 3100 3110 3120
CCTTGTGGCA ATGATAAAAA GAAATTTTAA CTCTCCAGAT
GGAACACCGT TACTATTTTT CTTTAAAATT GAGAGGTCTA

3130 3140 3150 3160
TTAACGGGTA TATTAGATAT TGAAGATACT GCCGAACTTG
AATTGCCCAT ATAATCTATA ACTTCTATGA CGGCTTGAAC

3170 3180 3190 3200
TAGTTAATAA GTTTTGGGAT GCTTACATTA TTGACGAACT
ATCAATTATT CAAAACCCTA CGAATGTAAT AACTGCTTGA

3210 3220 3230 3240
TTCTGGTGGA AATGTTACAC CGATGACTTC AGATGCCTTT
AAGACCACCT TTACAATGTG GCTACTGAAG TCTACGGAAA

3250 3260 3270 3280
CACAGGTGGA TGGCTAAGCA GGAGAAAAGT ACTATTGGTC
GTGTCCACCT ACCGATTCGT CCTCTTTTCA TGATAACCAG

3290 3300 3310 3320
AATTAGCTGA TTTTGACTTT GTTGATTTAC CCGCTATTGA
TTAATCGACT AAAACTGAAA CAACTAAATG GGCGATAACT

3330 3340 3350 3360
TCAATACAAA CATATGATTA AGGCGCAACC TAAACAGAAG
AGTTATGTTT GTATACTAAT TCCGCGTTGG ATTTGTCTTC

3370 3380 3390 3400
CTTGATCTCA GTCCTCAAGA TGAGTATGCT GCACTTCAGA
GAACTAGAGT CAGGAGTTCT ACTCATACGA CGTGAAGTCT

3410 3420 3430 3440
CTATTGTTTA TCACTCAAAG CAGATAAATG CAATTTTCGG
GATAACAAAT AGTGAGTTTC GTCTATTTAC GTTAAAAGCC

3450 3460 3470 3480
TCCTCTTTTT TCCGAATTAA CTAGACAGCT TTTAGAGAGG
AGGAGAAAAA AGGCTTAATT GATCTGTCGA AAATCTCTCC

3490 3500 3510 3520
ATAGACAGTA GCAAATTTTT GTTTTACACC CGGAAAACGC
TATCTGTCAT CGTTTAAAAA CAAAATGTGG GCCTTTTGCG

3530 3540 3550 3560
CCGAACAGAT TGAAGAGTTT TTCTCAGATT TGGATTCGAC
GGCTTGTCTA ACTTCTCAAA AAGAGTCTAA ACCTAAGCTG

3570 3580 3590 3600
GGTGCCAATG GAAGTTCTAG AGTTAGACAT TTCAAAGTAT
CCACGGTTAC CTTCAAGATC TCAATCTGTA AAGTTTCATA

3610 3620 3630 3640
GACAAATCCC AGAACGAATT CCATTGTGCT GTTGAGTACT
CTGTTTAGGG TCTTGCTTAA GGTAACACGA CAACTCATGA

3650 3660 3670 3680
TGATTTGGGA GAAACTTGGT CTTAATGGCT TTCTCGAAGA
ACTAAACCCT CTTTGAACCA GAATTACCGA AAGAGCTTCT

3690 3700 3710 3720
AGTTTGGAAA CAGGGACATA GGAAGACATC CTTGAAGGAT
TCAAACCTTT GTCCCTGTAT CCTTCTGTAG GAACTTCCTA

3730 3740 3750 3760
TACACCGCAG GAATAAAGAC TTGTTTATGG TATCAAAGGA
ATGTGGCGTC CTTATTTCTG AACAAATACC ATAGTTTCCT

3770 3780 3790 3800
AAAGCGGAGA TGTAACTACA TTTATTGGCA ATACAGTTAT
TTTCGCCTCT ACATTGATGT AAATAACCGT TATGTCAATA

3810 3820 3830 3840
TATAGCTGCT TGTCTTGCGT CGATGATACC TATGGATAAG
ATATCGACGA ACAGAACGCA GCTACTATGG ATACCTATTC

3850 3860 3870 3880
GTAATTAAAG CTGCGTTTTG TGGAGATGAT AGTATGCTAT
CATTAATTTC GACGCAAAAC ACCTCTACTA TCATACGATA

3890 3900 3910 3920
ACATTCCAAA GGGACTTGAT CTTCCTGATA TCCAATCTGG
TGTAAGGTTT CCCTGAACTA GAAGGACTAT AGGTTAGACC

3930 3940 3950 3960
AGCGAATTTG ATGTGGAACT TCGAGGCAAA ATTATATCGT
TCGCTTAAAC TACACCTTGA AGCTCCGTTT TAATATAGCA

3970 3980 3990 4000
AAGCGTTACG GATATTTTTG CGGGAGGTAT ATTATTCATC
TTCGCAATGC CTATAAAAAC GCCCTCCATA TAATAAGTAG

4010 4020 4030 4040
ATGACAGAGG TGCCATTGTG TATTACGACC CTCTTAAATT
TACTGTCTCC ACGGTAACAC ATAATGCTGG GAGAATTTAA

4050 4060 4070 4080
AATTTCTAAG TTAGGTTGTA AACACATCAA ATCTTTGGAT
TTAAAGATTC AATCCAACAT TTGTGTAGTT TAGAAACCTA

4090 4100 4110 4120
CATTTGGAAG AATTTAGAAT CTCGCTGTGT GACGTAAGCG
GTAAACCTTA TTAAATCTTA GAGCGACACA CTGCATTCGC

4130 4140 4150 4160
CTTCTTTAAA TAATTGTGCG TATTTTGGTC AATTGAATGA
GAAGAAATTT ATTAACACGC ATAAAACCAG TTAACTTACT

4170 4180 4190 4200
TGCAATTGCT GAGGTCCACA AGACCGCAGT AAATGGGTCG
ACGTTAACGA CTCCAGGTGT TCTGGCGTCA TTTACCCAGC

4210 4220 4230 4240
TTTGCGTTTT GTAGTATTGT TAAGTATCTT TCCGATAAAA
AAACGCAAAA CATCATAACA ATTCATAGAA AGGCTATTTT

4250 4260 4270 4280
ACCTTTTCAG AACCTTGTTC TACAATGGCT CTAGTACTAA
TGGAAAAGTC TTGGAACAAG ATGTTACCGA GATCATGATT

4290 4300 4310 4320
GGGATAGCAT CAAAATCTCT GAATTTATTA ATTTATCAGC
CCCTATCGTA GTTTTAGAGA CTTAAATAAT TAAATAGTCG

4330 4340 4350 4360
CTCTGAAAAA CTACTACCGT CAGCTCTAAC GGCTGTCAAG
GAGACTTTTT GATGATGGCA GTCGAGATTG CCGACAGTTC

4370 4380 4390 4400
TCGGTACGTA TTTCAAAGGT TGATAAAATT ATAAGTTACG
AGCCATGCAT AAAGTTTCCA ACTATTTTAA TATTCAATGC

4410 4420 4430 4440
AGAATGATAC TTTGTCTGAT ATTGATTTGC TTAAAGGTGT
TCTTACTATG AAACAGACTA TAACTAAACG AATTTCCACA

4450 4460 4470 4480
GAAGTTAGTA GAGAATGGGT ATGTGTGCCT TGCCGGCCTC
CTTCAATCAT CTCTTACCCA TACACACGGA ACGGCCGGAG

4490 4500 4510 4520
GTCGTCACTG GTGAATGGAA TTTACCGGAC AATTGCAAAG
CAGCAGTGAC CACTTACCTT AAATGGCCTG TTAACGTTTC

4530 4540 4550 4560
GTGGTGTGAG TATCTGTCTT GTAGACAAAC GCATGAAAAG
CACCACACTC ATAGACAGAA CATCTGTTTG CGTACTTTTC

4570 4580 4590 4600
AGCTAATGAA GCAACACTTG GCTCTTATCA TACATCTGCT
TCGATTACTT CGTTGTGAAC CGAGAATAGT ATGTAGACGA

4610 4620 4630 4640
TGCAAGAAGA GATTCACCTT TAAAATTATC CCGAATTACA
ACGTTCTTCT CTAAGTGGAA ATTTTAATAG GGCTTAATGT

4650 4660 4670 4680
GTGTCACCAC TGCCGACGCT CTTAAAGGAA TATGGCAAGT
CACAGTGGTG ACGGCTGCGA GAATTTCCTT ATACCGTTCA

4690 4700 4710 4720
TATGACAAAT ATTAGAGGTG TGGAAATGGA AAAGGGTTTT
ATACTGTTTA TAATCTCCAC ACCTTTACCT TTTCCCAAAA

4730 4740 4750 4760
TGCCCATTAT CTTTGGAGTT TGTTTCTATT TGCGTGGTGT
ACGGGTAATA GAAACCTCAA ACAAAGATAA ACGCACCACA

4770 4780 4790 4800
ATTTAAATAA TATAAAACTC GGACTGAGAG AGAAGATTTT
TAAATTTATT ATATTTTGAG CCTGACTCTC TCTTCTAAAA

4810 4820 4830 4840
GAACGTAACA GAAGGAGGGC CCACCGAACT TACTGAAGCA
CTTGCATTGT CTTCCTCCCG GGTGGCTTGA ATGACTTCGT

4850 4860 4870 4880
GTTGTTGATG AGTTCGTGGA GAAAGTTCCT ATGGCTGCTA
CAACAACTAC TCAAGCACCT CTTTCAAGGA TACCGACGAT

4890 4900 4910 4920
GGCTCAAGTC TTTTCGCTCT GTCAACAAAA AGAAACCTAG
CCGAGTTCAG AAAAGCGAGA CAGTTGTTTT TCTTTGGATC

4930 4940 4950 4960
TAACAGTTCT AAGTTTGTTA ATGGAAAATC TAGACTTAAT
ATTGTCAAGA TTCAAACAAT TACCTTTTAG ATCTGAATTA

4970 4980 4990 5000
AGTCGTAATA AATTAAATTA TGAAAATGGT GATAGTGATG
TCAGCATTAT TTAATTTAAT ACTTTTACCA CTATCACTAC

5010 5020 5030 5040
TTGGTATTAG TGTTGTAGAT GATATTGTGG TTGGTAATGG
AACCATAATC ACAACATCTA CTATAACACC AACCATTACC

5050 5060 5070 5080
TGTTAGTGAT ATTCGTATTG ATGATGATTG TGAGTCATTT
ACAATCACTA TAAGCATAAC TACTACTAAC ACTCAGTAAA

5090 5100 5110 5120
GACGCACAAT CTGATTCGTA TTGAATATGT CTTACACTAT
CTGCGTGTTA GACTAAGCAT AACTTATACA GAATGTGATA

5130 5140 5150 5160
TACAGACCCG TCTAAGCTGG CTTATTTAAG CTCGGCTTGG
ATGTCTGGGC AGATTCGACC GAATAAATTC GAGCCGAACC

5170 5180 5190 5200
GCTGACCCCA ATTCACTGAT CAACCTTTGT ACCAATTCTC
CGACTGGGGT TAAGTGACTA GTTGGAAACA TGGTTAAGAG

5210 5220 5230 5240
TGGGTAATCA GTTCCAAACA CAACAAGCTC GAACAACTGT
ACCCATTAGT CAAGGTTTGT GTTGTTCGAG CTTGTTGACA

5250 5260 5270 5280
TCAACAGCAG TTTGCTGATG TTTGGCAGCC GGTTCCTACT
AGTTGTCGTC AAACGACTAC AAACCGTCGG CCAAGGATGA

5290 5300 5310 5320
TTGACCAGTA GGTTCCCTGC AGGCGCTGGT TACTTCAGAG
AACTGGTCAT CCAAGGGACG TCCGCGACCA ATGAAGTCTC

5330 5340 5350 5360
TTTATCGCTA TGATCCTATA TTAGATCCTT TAATAACTTT
AAATAGCGAT ACTAGGATAT AATCTAGGAA ATTATTGAAA

5370 5380 5390 5400
CTTAATGGGT ACTTTTGATA CTCGTAATAG AATAATCGAG
GAATTACCCA TGAAAACTAT GAGCATTATC TTATTAGCTC

5410 5420 5430 5440
GTAGAAAATC CGCAGAATCC GACAACTACG GAAACATTAG
CATCTTTTAG GCGTCTTAGG CTGTTGATGC CTTTGTAATC

5450 5460 5470 5480
ATGCAACTCG TAGAGTTGAT GATGCAACTG TAGCAATAAG
TACGTTGAGC ATCTCAACTA CTACGTTGAC ATCGTTATTC

5490 5500 5510 5520
ATCTGCAATA AATAATCTAT TAAATGAGTT AGTTAGGGGA
TAGACGTTAT TTATTAGATA ATTTACTCAA TCAATCCCCT

5530 5540 5550 5560
ACTGGTATGT ACAATCAAGT CTCATTTGAG ACGATATCTG
TGACCATACA TGTTAGTTCA GAGTAAACTC TGCTATAGAC

5570 5580 5590 5600
GACTTACTTG GACCTCTTCC TAATCATATT TAGGAAAATA
CTGAATGAAC CTGGAGAAGG ATTAGTATAA ATCCTTTTAT

5610 5620 5630 5640
ACGTTGATAG TGTTGAACTA TCCGTGGTGC ATACGATAAT
TGCAACTATC ACAACTTGAT AGGCACCACG TATGCTATTA

5650 5660 5670 5680
GCATAGTGGT TATCCCTCCA CTTAAATCGA AGGGTTTTTC
CGTATCACCA ATAGGGAGGT GAATTTAGCT TCCCAAAAAG

5690 5700 5710 5720
ACTGCAGATA TGTAGGTTTC CTACGGTGAA TATAAAACTT
TGACGTCTAT ACATCCAAAG GATGCCACTT ATATTTTGAA

5730 5740 5750 5760
ATATCCCGTT GTGTACACGA TAGTACATAG TGTTTATCCC
TATAGGGCAA CACATGTGCT ATCATGTATC ACAAATAGGG

5770 5780 5790 5800
TCCACTTAAA TCGAAGGGTT TTGTGTCAGA CGCGTGTAAG
AGGTGAATTT AGCTTCCCAA AACACAGTCT GCGCACATTC

5810 5820 5830 5840
GAGTGGTCAA CCTTACGACA CATTTAAAAT AATGCGTCCG
CTCACCAGTT GGAATGCTGT GTAAATTTTA TTACGCAGGC

5850 5860 5870 5880
TGGTGCATAC GATAATGCAT AGTGTTTGTC CCTCCACTTA
ACCACGTATG CTATTACGTA TCACAAACAG GGAGGTGAAT

5890 5900 5910 5920
AATCGAAGGG TTGTGTATAT GGATCATGCG GATAAAGTTA
TTAGCTTCCC AACACATATA CCTAGTACGC CTATTTCAAT

5930 5940 5950 5960
TACTGGTGCA GTATAACCCG TTATACACAT AAAATTATGA
ATGACCACGT CATATTGGGC AATATGTGTA TTTTAATACT

5970 5980 5990
GGGATTCGAA TTCCCCCTTA CCTCGGGTAG AGGCCCA 3′
CCCTAAGCTT AAGGGGGAAT GGAGCCCATC TCCGGGT 5′

3. A DNA sequence capable of hybridising with a DNA sequence according to claim 1 or 2 under the following conditions.
(i) A DNA sequence is fixed on a nitrocellulose filter and the filter is equilibrated in a buffer containing 25mM KPO₄ (pH 7.4), 5 x SSC, 5 x Deuhardt solution (containing 0.2% phycol (molecular weight: 40,000), 0.2% polyvinyl pyrrolidine (molecular weight: 30,000 - 40,000) and 0.2% bovine serum albumin (fraction v), 50µg/ml salmon sperm DNA and 50% formaldehyde, at 37-42°C for 3 hours overnight.
(ii) the filter is subsequently equilibrated in the same buffer modified by further containing 10% dextran sulphate and a DNA sequence according to Claim 1 or 2 at 37-42°C for 6 hours to overnight; and
(iii) the filter is subsequently assayed for hybridisation between fixed DNA and DNA according to Claim 1 and 2.

4. A method for producing DNA according to any of Claims 1-3 which comprises isolating an Odontoglossum ringspot virus RNA gene and producing cDNA complementary to said isolated viral RNA.

5. A method for producing DNA according to Claim 4 which comprises further cloning the resulting cDNA.

6. A recombinant vector which comprises isolated DNA according to any of Claims 1-3.

7. A transformant which comprises a recombinant vector according to Claim 6.

8. A transformant according to Claim 7 wherein the host cell is selected from the group consisting of E.Coli, Bacillus, yeast, Agrobacterium, and plant cells.

9. A method for producing a transformant which comprises transforming a host cell with a recombinant vector according to Claim 6.

10. A method for assaying an Odontoglossum ringspot virus-related gene which comprises hybridizing a nucleic acid selected from the group consisting of Odontoglossum ringspot virus RNA genes, and cDNA obtained by reverse-transcripting said virus RNA gene or cloning, with a probe consisting of isolated DNA according to any of Claims 1-3 and detecting a hybridized product.

11. A 33K protein or coat protein, produced by expression of Recombinant DNA, of Odontoglossum ringspot virus.

12. A peptide fragment for use as a reagent for screening Odontoglossum ringspot virus genes characterised in that the peptide fragment is obtained by expression of Recombinant DNA having a sequence according to Claim 1 or 3.

## Patentansprüche

1. Isolierte DNA-Sequenz, entsprechend (1) einem Genom des Odontoglossum Ringspot Virus (ORSV) oder einem Fragment derselben, welches durch Spaltung mit einer oder mehreren Restriktionsendonukleasen ausgewählt aus EcoRI, AccI, PstI, HindIII, HpaI, MluI, XbaI und ApaI erzeugt wird, oder entsprechend (2) einer zu (1) komplementären Sequenz.

2. DNA-Sequenz gemäß Anspruch 1, bei der das ORSV-Genom die Sequenz hat:

10 20 30 40
5′ GACTGTTCTT ACTCCGTTGA TCTTCCGGGA AAAACATACG
3′ CTGACAAGAA TGAGGCAACT AGAAGGCCCT TTTTGTATGC

50 60 70 80
CTGTGGCGCT TCATAGTATC TATGATATTC CAGCGGATGA
GACACCGCGA AGTATCATAG ATACTATAAG GTCGCCTACT

90 100 110 120
GTTCGGAGCT GCCTTATTAC GAAAGGATGT TCACATATGT
CAAGCCTCGA CGGAATAATG CTTTCCTACA AGTGTATACA

130 140 150 160
TATGCTGCTT TTCATTTTAG TGAGAATCTG CTTCTTGAGA
ATACGACGAA AAGTAAAATC ACTCTTAGAC GAAGAACTCT

170 180 190 200
CTACGTCTGC TCCGCTCGAT GAGATTGGAG CGACGTTTTA
GATGCAGACG AGGCGAGCTA CTCTAACCTC GCTGCAAAAT

210 220 230 240
CAAGTCAGGG GATAGATTAT CCTTCTTCTT TCAAAATGAG
GTTCAGTCCC CTATCTAATA GGAAGAAGAA AGTTTTACTC

250 260 270 280
AGCACATTGA ATTATGAACA TTCTTATAAA AACGTAATTA
TCGTGTAACT TAATACTTGT AAGAATATTT TTGCATTAAT

290 300 310 320
AATATGTTTG CAAGACTTTT TTCCCAGCTT CAAATAGGTT
TTATACAAAC GTTCTGAAAA AAGGGTCGAA GTTTATCCAA

330 340 350 360
CGTCTACCAT AAGGAATTTA TGTGCACCAG AGTTAACACA
GCAGATGGTA TTCCTTAAAT ACACGTGGTC TCAATTGTGT

850 860 870 880
GATCAAGTCG TACTTAAAAA ATTTCAAAAA TTTGACGATA
CTAGTTCAGC ATGAATTTTT TAAAGTTTTT AAACTGCTAT

890 900 910 920
CAGTTACTAA TCTTTTCTGG AAACAGATTA GTGATGCTGT
GTCAATGATT AGAAAAGACC TTTGTCTAAT CACTACGACA

930 940 950 960
AGGCGATCTC TTCCCGTCGA TTAAGGAAAG GCTCATTAGC
TCCGCTAGAG AAGGGCAGCT AATTCCTTTC CGAGTAATCG

970 980 990 1000
GGTGGGTTTG TAAAAGTTGC TGAGCAATCT TTACAAATTA
CCACCCAAAC ATTTTCAACG ACTCGTTAGA AATGTTTAAT

1010 1020 1030 1040
AAACGCCTGA TGAGTATATC ACCTTTGCAG ATAAACTGGT
TTTGCGGACT ACTCATATAG TGGAAACGTC TATTTGACCA

1050 1060 1070 1080
TATGGAATAT AAAGCAACTG AAGAGCTTCA ACATCTAGAC
ATACCTTATA TTTCGTTGAC TTCTCGAAGT TGTAGATCTG

1090 1100 1110 1120
ATTAGTAAGC CTCTTGAGAG AGCCGAGAAA TATTATAATG
TAATCATTCG GAGAACTCTC TCGGCTCTTT ATAATATTAC

1130 1140 1150 1160
CTTTATCGGA ACTATCGGTT CTCAAGGAAT GTGACGAGTT
GAAATAGCCT TGATAGCCAA GAGTTCCTTA CACTGCTCAA

1170 1180 1190 1200
TGATATAACG CAATTCAAGA ATTTATGTGA AGAAAAGGAC
ACTATATTGC GTTAAGTTCT TAAATACACT TCTTTTCCTG

1210 1220 1230 1240
ATTGATCCAG ACGTCGTGGC GAAAGTGATC GTCGCCATCA
TAACTAGGTC TGCAGCACCG CTTTCACTAG CAGCGGTAGT

1250 1260 1270 1280
TGAAGAACGA ACTCACACTG CCTTTTAAAA ATCCAACGCC
ACTTCTTGCT TGAGTGTGAC GGAAAATTTT TAGGTTGCGG

1290 1300 1310 1320
TGAAGCATTA TCGGATGCTC TTTCACCGTT ACCCAAAGAC
ACTTCGTAAT AGCCTACGAG AAAGTGGCAA TGGGTTTCTG

370 380 390 400
TGGTTTTGTA AGTTCACCAA GGTAGATACT TATTTCCTTT
ACCAAAACAT TCAAGTGGTT CCATCTATGA ATAAAGGAAA

410 420 430 440
TCCGAGGGGT GTATACTAGG GGAGAAGATT CTGAACAATT
AGGCTCCCCA CATATGATCC CCTCTTCTAA GACTTGTTAA

450 460 470 480
TTACACTGCT ATGGATGAAG CATGGGAGTA TAAGAAAACT
AATGTGACGA TACCTACTTC GTACCCTCAT ATTCTTTTGA

490 500 510 520
TTGGCAATGC TTAATAGCGA AAGGACGATC TTTCGCGATC
AACCGTTACG AATTATCGCT TTCCTGCTAG AAAGCGCTAG

530 540 550 560
GCGCTGCGGT TAACTTTTGG TTTCCTAAAG TTAAGGATAT
CGCGACGCCA ATTGAAAACC AAAGGATTTC AATTCCTATA

570 580 590 600
GGTTATTGTG CCGCTTTTTG ATGGTTCCGT CACTTCAGGA
CCAATAACAC GGCGAAAAAC TACCAAGGCA GTGAAGTCCT

610 620 630 640
AAGATGAAAA GAAGTGAAGT CATGGTTAAC AAGGATTTTG
TTCTACTTTT CTTCACTTCA GTACCAATTG TTCCTAAAAC

650 660 670 680
TTTACACCGT TTTAAATCAC ATCCGTACCT ATCAAGACAA
AAATGTGGCA AAATTTAGTG TAGGCATGGA TAGTTCTGTT

690 700 710 720
AGCCTTGACG TATAAAAACG TTCTTTCCTT TGTCGAGTCA
TCGGAACTGC ATATTTTTGC AAGAAAGGAA ACAGCTCAGT

730 740 750 760
ATTAGGTCTC GTGTGATTAT TAATGGTGTG ACTGCAAGAT
TAATCCAGAG CACACTAATA ATTACCACAC TGACGTTCTA

770 780 790 800
CTGAGTGGGA TGTAGACAAG TCTGTGTTAC AGGCGTTATC
GACTCACCCT ACATCTGTTC AGACACAATG TCCGCAATAG

810 820 830 840
TATGACTTTT CTTTTACAGA CGAAGTTGGC TGAAGCAAAA
ATACTGAAAA GAAAATGTCT GCTTCAACCG ACTTCGTTTT

1330 1340 1350 1360
TTGGATATGA GATTTGATCT GCTTAAATTA AGTACGTGTG
AACCTATACT CTAAACTAGA CGAATTTAAT TCATGCACAC

1370 1380 1390 1400
CACCCTTTCC AAGCGTTAAA ACTTTAGATA GTGGTTTATT
GTGGGAAAGG TTCGCAATTT TGAAATCTAT CACCAAATAA

1410 1420 1430 1440
ACCAAAGCAA AGCTACGGTG ACGAAAGACA ATTTGAGAGT
TGGTTTCGTT TCGATGCCAC TGCTTTCTGT TAAACTCTCA

1450 1460 1470 1480
CAATCTGTCG TGTCTGTATC TGATTTTCAT CTTAAGAGCG
GTTAGACAGC ACAGACATAG ACTAAAAGTA GAATTCTCGC

1490 1500 1510 1520
TCGAGTCAGT AAAAATGAAA AGTATGTCGT CAGCGGTCTA
AGCTCAGTCA TTTTTACTTT TCATACAGCA GTCGCCAGAT

1530 1540 1550 1560
CACAGGTCCC CTCAAAGTTC AACAGATGAA GAACTATATG
GTGTCCAGGG GAGTTTCAAG TTGTCTACTT CTTGATATAC

1570 1580 1590 1600
GATTACCTGT CCGCATCGAT TTCGGCTACT GTTTCAAATT
CTAATGGACA GGCGTAGCTA AAGCCGATGA CAAAGTTTAA

1610 1620 1630 1640
TATGCAAGGT GCTAAAGGAT GTCTATGGTG CCGACCCAGA
ATACGTTCCA CGATTTCCTA CAGATACCAC GGCTGGGTCT

1650 1660 1670 1680
GTCTGCAGAA AAGTCTGGAG TCTACGATGT AGTGAAAGGC
CAGACGTCTT TTCAGACCTC AGATGCTACA TCACTTTCCG

1690 1700 1710 1720
AAATGGTTAT TAAAACCAAA GGGTAAATGT CACGCTTGGG
TTTACCAATA ATTTTGGTTT CCCATTTACA GTGCGAACCC

1730 1740 1750 1760
GTGTTGCCGA ACTTAACAAT GGCGAGAAAG TCATCGTATT
CACAACGGCT TGAATTGTTA CCGCTCTTTC AGTAGCATAA

1770 1780 1790 1800
ACTCGAATGG GCTGATGGAT TCCCTATTTG TGGTGATTGG
TGAGCTTACC CGACTACCTA AGGGATAAAC ACCACTAACC

1810 1820 1830 1840
CGACGAGTGG CCGTCTCTTC AGATTCCCTT ATTTATTCAG
GCTGCTCACC GGCAGAGAAG TCTAAGGGAA TAAATAAGTC

1850 1860 1870 1880
ATATGGGAAA ACTTCAGACG TTGTTAAGCT GCTTAAAGGA
TATACCCTTT TGAAGTCTGC AACAATTCGA CGAATTTCCT

1890 1900 1910 1920
CGGTGAGCCT GTACCTTCGG ATGCGAAAGT CACGTTAGTA
GCCACTCGGA CATGGAAGCC TACGCTTTCA GTGCAATCAT

1930 1940 1950 1960
GATGGCGTTC CTGGTTGTGG AAAAACAAAG GAAATTCTTG
CTACCGCAAG GACCAACACC TTTTTGTTTC CTTTAAGAAC

1970 1980 1990 2000
AAACTGTGAA TTTTGATGAG GACTTAATAC TTGTTCCTGG
TTTGACACTT AAAACTACTC CTGAATTATG AACAAGGACC

2010 2020 2030 2040
TAAAGAAGCT TGTAAAATGA TTATCAAGAG AGCTAATAAG
ATTTCTTCGA ACATTTTACT AATAGTTCTC TCGATTATTC

2050 2060 2070 2080
TCTGGACATG TAAGAGCGAC AAAGGATAAT GTAAGAACTG
AGACCTGTAC ATTCTCGCTG TTTCCTATTA CATTCTTGAC

2090 2100 2110 2120
TGGATTCGTT CTTAATGCAT TTGAAGCCTA AGACTTATAA
ACCTAAGCAA GAATTACGTA AACTTCGGAT TCTGAATATT

2130 2140 2150 2160
CAAACTGTTT ATCGATGAGG GTTTGATGTT ACATACTGGT
GTTTGACAAA TAGCTACTCC CAAACTACAA TGTATGACCA

2170 2180 2190 2200
TGTGTTAACT TTCTGATAGC ATTGTCCCAT TGTCGTGAAG
ACACAATTGA AAGACTATCG TAACAGGGTA ACAGCACTTC

2210 2220 2230 2240
CAATGGTTTT TGGTGATACT GAACAAATTC CCTTTATAAA
GTTACCAAAA ACCACTATGA CTTGTTTAAG GGAAATATTT

2250 2260 2270 2280
CAGAGTGGCA AATTTCCCTT ATCCGAAACA TTTTGCCACA
GTCTCACCGT TTAAAGGGAA TAGGCTTTGT AAAACGGTGT

2290 2300 2310 2320
CTTGTTTATG ATCATAGGGA GGTTAGGAGG TTATCACTTA
GAACAAATAC TAGTATCCCT CCAATCCTCC AATAGTGAAT

2330 2340 2350 2360
GATGCCCGGC AGATGTTACG CATTTTATGA ACTCCAAATA
CTACGGGCCG TCTACAATGC GTAAAATACT TGAGGTTTAT

2370 2380 2390 2400
TGACGGGAAA GTTTTATGTA CGAATGATGT GATTCGCTCA
ACTGCCCTTT CAAAATACAT GCTTACTACA CTAAGCGAGT

2410 2420 2430 2440
GTTGATGCTG AAGTGGTTAG AGGGAAAGGG GTTTTTAACC
CAACTACGAC TTCACCAATC TCCCTTTCCC CAAAAATTGG

2450 2460 2470 2480
CGAAAAGCAA ACCGCTTAAA GGGAAAATTA TCACCTTCAC
GCTTTTCGTT TGGCGAATTT CCCTTTTAAT AGTGGAAGTG

2490 2500 2510 2520
TCAAAGTGAC AAAGCTGAAC TGAAGGAGCG TGGTTATGAA
AGTTTCACTG TTTCGACTTG ACTTCCTCGC ACCAATACTT

2530 2540 2550 2560
GAAGTTTCAA CTTTTGGTGA AATTAACACC GTACACGAAA
CTTCAAAGTT GAAAACCACT TTAATTGTGG CATGTGCTTT

2570 2580 2590 2600
TCCAAGGTGA GACCTTTGAA GACGTATCGG TTGTTCGTTT
AGGTTCCACT CTGGAAACTT CTGCATAGCC AACAAGCAAA

2610 2620 2630 2640
GACACCAACT CCATTGGAGC TTATATCAAA GAGTTCACCG
CTGTGGTTGA GGTAACCTCG AATATAGTTT CTCAAGTGGC

2650 2660 2670 2680
CACGTTCTTG TTGCATTGAC AAGACATACT AAAAGCTTCA
GTGCAAGAAC AACGTAACTG TTCTGTATGA TTTTCGAAGT

2690 2700 2710 2720
AATATTACTC TGTGGTTCTC GATCCTCTCG TCAAAGTTTG
TTATAATGAG ACACCAAGAG CTAGGAGAGC AGTTTCAAAC

2730 2740 2750 2760
CTCTGATTTA AGCAAGGTGT CAGACTTTAT ACTGGATATG
GAGACTAAAT TCGTTCCACA GTCTGAAATA TGACCTATAC

2770 2780 2790 2800
TATAAGGTCG ATGCCGGGAT CTTATAGCAA TTACAGGTAG
ATATTCCAGC TACGGCCCTA GAATATCGTT AATGTCCATC

2810 2820 2830 2840
GGAGTATCTT CAAAGGCGAA AATCTATTCG TACCATGTCC
CCTCATAGAA GTTTCCGCTT TTAGATAAGC ATGGTACAGG

2850 2860 2870 2880
TAAATCTGGT TATATTTCTG ATATGCAATT TTATTATGAC
ATTTAGACCA ATATAAAGAC TATACGTTAA AATAATACTG

2890 2900 2910 2920
ACTTTGTTAC CTGGAAACAG CACTATTTTA AACGAGTATG
TGAAACAATG GACCTTTGTC GTGATAAAAT TTGCTCATAC

2930 2940 2950 2960
ATGCAGTGAC TATGAATCTT CGTGAAAATA ATCTTAATGT
TACGTCACTG ATACTTAGAA GCACTTTTAT TAGAATTACA

2970 2980 2990 3000
CAAGGATTGC ACGATAGATT TTTCTAAATC TGTTAGTGTC
GTTCCTAACG TGCTATCTAA AAAGATTTAG ACAATCACAG

3010 3020 3030 3040
CCGAGACAAC AACAAGAGTT TTTCACACCA GTTATTCGAA
GGCTCTGTTG TTGTTCTCAA AAAGTGTGGT CAATAAGCTT

3050 3060 3070 3080
CTGCTGCTGA ACGTCCACGT AGTCGTGGTC TCCTTGAAAA
GACGACGACT TGCAGGTGCA TCAGCACCAG AGGAACTTTT

3090 3100 3110 3120
CCTTGTGGCA ATGATAAAAA GAAATTTTAA CTCTCCAGAT
GGAACACCGT TACTATTTTT CTTTAAAATT GAGAGGTCTA

3130 3140 3150 3160
TTAACGGGTA TATTAGATAT TGAAGATACT GCCGAACTTG
AATTGCCCAT ATAATCTATA ACTTCTATGA CGGCTTGAAC

3170 3180 3190 3200
TAGTTAATAA GTTTTGGGAT GCTTACATTA TTGACGAACT
ATCAATTATT CAAAACCCTA CGAATGTAAT AACTGCTTGA

3210 3220 3230 3240
TTCTGGTGGA AATGTTACAC CGATGACTTC AGATGCCTTT
AAGACCACCT TTACAATGTG GCTACTGAAG TCTACGGAAA

3250 3260 3270 3280
CACAGGTGGA TGGCTAAGCA GGAGAAAAGT ACTATTGGTC
GTGTCCACCT ACCGATTCGT CCTCTTTTCA TGATAACCAG

3290 3300 3310 3320
AATTAGCTGA TTTTGACTTT GTTGATTTAC CCGCTATTGA
TTAATCGACT AAAACTGAAA CAACTAAATG GGCGATAACT

3330 3340 3350 3360
TCAATACAAA CATATGATTA AGGCGCAACC TAAACAGAAG
AGTTATGTTT GTATACTAAT TCCGCGTTGG ATTTGTCTTC

3370 3380 3390 3400
CTTGATCTCA GTCCTCAAGA TGAGTATGCT GCACTTCAGA
GAACTAGAGT CAGGAGTTCT ACTCATACGA CGTGAAGTCT

3410 3420 3430 3440
CTATTGTTTA TCACTCAAAG CAGATAAATG CAATTTTCGG
GATAACAAAT AGTGAGTTTC GTCTATTTAC GTTAAAAGCC

3450 3460 3470 3480
TCCTCTTTTT TCCGAATTAA CTAGACAGCT TTTAGAGAGG
AGGAGAAAAA AGGCTTAATT GATCTGTCGA AAATCTCTCC

3490 3500 3510 3520
ATAGACAGTA GCAAATTTTT GTTTTACACC CGGAAAACGC
TATCTGTCAT CGTTTAAAAA CAAAATGTGG GCCTTTTGCG

3530 3540 3550 3560
CCGAACAGAT TGAAGAGTTT TTCTCAGATT TGGATTCGAC
GGCTTGTCTA ACTTCTCAAA AAGAGTCTAA ACCTAAGCTG

3570 3580 3590 3600
GGTGCCAATG GAAGTTCTAG AGTTAGACAT TTCAAAGTAT
CCACGGTTAC CTTCAAGATC TCAATCTGTA AAGTTTCATA

3610 3620 3630 3640
GACAAATCCC AGAACGAATT CCATTGTGCT GTTGAGTACT
CTGTTTAGGG TCTTGCTTAA GGTAACACGA CAACTCATGA

3650 3660 3670 3680
TGATTTGGGA GAAACTTGGT CTTAATGGCT TTCTCGAAGA
ACTAAACCCT CTTTGAACCA GAATTACCGA AAGAGCTTCT

3690 3700 3710 3720
AGTTTGGAAA CAGGGACATA GGAAGACATC CTTGAAGGAT
TCAAACCTTT GTCCCTGTAT CCTTCTGTAG GAACTTCCTA

3730 3740 3750 3760
TACACCGCAG GAATAAAGAC TTGTTTATGG TATCAAAGGA
ATGTGGCGTC CTTATTTCTG AACAAATACC ATAGTTTCCT

3770 3780 3790 3800
AAAGCGGAGA TGTAACTACA TTTATTGGCA ATACAGTTAT
TTTCGCCTCT ACATTGATGT AAATAACCGT TATGTCAATA

3810 3820 3830 3840
TATAGCTGCT TGTCTTGCGT CGATGATACC TATGGATAAG
ATATCGACGA ACAGAACGCA GCTACTATGG ATACCTATTC

3850 3860 3870 3880
GTAATTAAAG CTGCGTTTTG TGGAGATGAT AGTATGCTAT
CATTAATTTC GACGCAAAAC ACCTCTACTA TCATACGATA

3890 3900 3910 3920
ACATTCCAAA GGGACTTGAT CTTCCTGATA TCCAATCTGG
TGTAAGGTTT CCCTGAACTA GAAGGACTAT AGGTTAGACC

3930 3940 3950 3960
AGCGAATTTG ATGTGGAACT TCGAGGCAAA ATTATATCGT
TCGCTTAAAC TACACCTTGA AGCTCCGTTT TAATATAGCA

3970 3980 3990 4000
AAGCGTTACG GATATTTTTG CGGGAGGTAT ATTATTCATC
TTCGCAATGC CTATAAAAAC GCCCTCCATA TAATAAGTAG

4010 4020 4030 4040
ATGACAGAGG TGCCATTGTG TATTACGACC CTCTTAAATT
TACTGTCTCC ACGGTAACAC ATAATGCTGG GAGAATTTAA

4050 4060 4070 4080
AATTTCTAAG TTAGGTTGTA AACACATCAA ATCTTTGGAT
TTAAAGATTC AATCCAACAT TTGTGTAGTT TAGAAACCTA

4090 4100 4110 4120
CATTTGGAAG AATTTAGAAT CTCGCTGTGT GACGTAAGCG
GTAAACCTTA TTAAATCTTA GAGCGACACA CTGCATTCGC

4130 4140 4150 4160
CTTCTTTAAA TAATTGTGCG TATTTTGGTC AATTGAATGA
GAAGAAATTT ATTAACACGC ATAAAACCAG TTAACTTACT

4170 4180 4190 4200
TGCAATTGCT GAGGTCCACA AGACCGCAGT AAATGGGTCG
ACGTTAACGA CTCCAGGTGT TCTGGCGTCA TTTACCCAGC

4210 4220 4230 4240
TTTGCGTTTT GTAGTATTGT TAAGTATCTT TCCGATAAAA
AAACGCAAAA CATCATAACA ATTCATAGAA AGGCTATTTT

4250 4260 4270 4280
ACCTTTTCAG AACCTTGTTC TACAATGGCT CTAGTACTAA
TGGAAAAGTC TTGGAACAAG ATGTTACCGA GATCATGATT

4290 4300 4310 4320
GGGATAGCAT CAAAATCTCT GAATTTATTA ATTTATCAGC
CCCTATCGTA GTTTTAGAGA CTTAAATAAT TAAATAGTCG

4330 4340 4350 4360
CTCTGAAAAA CTACTACCGT CAGCTCTAAC GGCTGTCAAG
GAGACTTTTT GATGATGGCA GTCGAGATTG CCGACAGTTC

4370 4380 4390 4400
TCGGTACGTA TTTCAAAGGT TGATAAAATT ATAAGTTACG
AGCCATGCAT AAAGTTTCCA ACTATTTTAA TATTCAATGC

4410 4420 4430 4440
AGAATGATAC TTTGTCTGAT ATTGATTTGC TTAAAGGTGT
TCTTACTATG AAACAGACTA TAACTAAACG AATTTCCACA

4450 4460 4470 4480
GAAGTTAGTA GAGAATGGGT ATGTGTGCCT TGCCGGCCTC
CTTCAATCAT CTCTTACCCA TACACACGGA ACGGCCGGAG

4490 4500 4510 4520
GTCGTCACTG GTGAATGGAA TTTACCGGAC AATTGCAAAG
CAGCAGTGAC CACTTACCTT AAATGGCCTG TTAACGTTTC

4530 4540 4550 4560
GTGGTGTGAG TATCTGTCTT GTAGACAAAC GCATGAAAAG
CACCACACTC ATAGACAGAA CATCTGTTTG CGTACTTTTC

4570 4580 4590 4600
AGCTAATGAA GCAACACTTG GCTCTTATCA TACATCTGCT
TCGATTACTT CGTTGTGAAC CGAGAATAGT ATGTAGACGA

4610 4620 4630 4640
TGCAAGAAGA GATTCACCTT TAAAATTATC CCGAATTACA
ACGTTCTTCT CTAAGTGGAA ATTTTAATAG GGCTTAATGT

4650 4660 4670 4680
GTGTCACCAC TGCCGACGCT CTTAAAGGAA TATGGCAAGT
CACAGTGGTG ACGGCTGCGA GAATTTCCTT ATACCGTTCA

4690 4700 4710 4720
TATGACAAAT ATTAGAGGTG TGGAAATGGA AAAGGGTTTT
ATACTGTTTA TAATCTCCAC ACCTTTACCT TTTCCCAAAA

4730 4740 4750 4760
TGCCCATTAT CTTTGGAGTT TGTTTCTATT TGCGTGGTGT
ACGGGTAATA GAAACCTCAA ACAAAGATAA ACGCACCACA

4770 4780 4790 4800
ATTTAAATAA TATAAAACTC GGACTGAGAG AGAAGATTTT
TAAATTTATT ATATTTTGAG CCTGACTCTC TCTTCTAAAA

4810 4820 4830 4840
GAACGTAACA GAAGGAGGGC CCACCGAACT TACTGAAGCA
CTTGCATTGT CTTCCTCCCG GGTGGCTTGA ATGACTTCGT

4850 4860 4870 4880
GTTGTTGATG AGTTCGTGGA GAAAGTTCCT ATGGCTGCTA
CAACAACTAC TCAAGCACCT CTTTCAAGGA TACCGACGAT

4890 4900 4910 4920
GGCTCAAGTC TTTTCGCTCT GTCAACAAAA AGAAACCTAG
CCGAGTTCAG AAAAGCGAGA CAGTTGTTTT TCTTTGGATC

4930 4940 4950 4960
TAACAGTTCT AAGTTTGTTA ATGGAAAATC TAGACTTAAT
ATTGTCAAGA TTCAAACAAT TACCTTTTAG ATCTGAATTA

4970 4980 4990 5000
AGTCGTAATA AATTAAATTA TGAAAATGGT GATAGTGATG
TCAGCATTAT TTAATTTAAT ACTTTTACCA CTATCACTAC

5010 5020 5030 5040
TTGGTATTAG TGTTGTAGAT GATATTGTGG TTGGTAATGG
AACCATAATC ACAACATCTA CTATAACACC AACCATTACC

5050 5060 5070 5080
TGTTAGTGAT ATTCGTATTG ATGATGATTG TGAGTCATTT
ACAATCACTA TAAGCATAAC TACTACTAAC ACTCAGTAAA

5090 5100 5110 5120
GACGCACAAT CTGATTCGTA TTGAATATGT CTTACACTAT
CTGCGTGTTA GACTAAGCAT AACTTATACA GAATGTGATA

5130 5140 5150 5160
TACAGACCCG TCTAAGCTGG CTTATTTAAG CTCGGCTTGG
ATGTCTGGGC AGATTCGACC GAATAAATTC GAGCCGAACC

5170 5180 5190 5200
GCTGACCCCA ATTCACTGAT CAACCTTTGT ACCAATTCTC
CGACTGGGGT TAAGTGACTA GTTGGAAACA TGGTTAAGAG

5210 5220 5230 5240
TGGGTAATCA GTTCCAAACA CAACAAGCTC GAACAACTGT
ACCCATTAGT CAAGGTTTGT GTTGTTCGAG CTTGTTGACA

5250 5260 5270 5280
TCAACAGCAG TTTGCTGATG TTTGGCAGCC GGTTCCTACT
AGTTGTCGTC AAACGACTAC AAACCGTCGG CCAAGGATGA

5290 5300 5310 5320
TTGACCAGTA GGTTCCCTGC AGGCGCTGGT TACTTCAGAG
AACTGGTCAT CCAAGGGACG TCCGCGACCA ATGAAGTCTC

5330 5340 5350 5360
TTTATCGCTA TGATCCTATA TTAGATCCTT TAATAACTTT
AAATAGCGAT ACTAGGATAT AATCTAGGAA ATTATTGAAA

5370 5380 5390 5400
CTTAATGGGT ACTTTTGATA CTCGTAATAG AATAATCGAG
GAATTACCCA TGAAAACTAT GAGCATTATC TTATTAGCTC

5410 5420 5430 5440
GTAGAAAATC CGCAGAATCC GACAACTACG GAAACATTAG
CATCTTTTAG GCGTCTTAGG CTGTTGATGC CTTTGTAATC

5450 5460 5470 5480
ATGCAACTCG TAGAGTTGAT GATGCAACTG TAGCAATAAG
TACGTTGAGC ATCTCAACTA CTACGTTGAC ATCGTTATTC

5490 5500 5510 5520
ATCTGCAATA AATAATCTAT TAAATGAGTT AGTTAGGGGA
TAGACGTTAT TTATTAGATA ATTTACTCAA TCAATCCCCT

5530 5540 5550 5560
ACTGGTATGT ACAATCAAGT CTCATTTGAG ACGATATCTG
TGACCATACA TGTTAGTTCA GAGTAAACTC TGCTATAGAC

5570 5580 5590 5600
GACTTACTTG GACCTCTTCC TAATCATATT TAGGAAAATA
CTGAATGAAC CTGGAGAAGG ATTAGTATAA ATCCTTTTAT

5610 5620 5630 5640
ACGTTGATAG TGTTGAACTA TCCGTGGTGC ATACGATAAT
TGCAACTATC ACAACTTGAT AGGCACCACG TATGCTATTA

5650 5660 5670 5680
GCATAGTGGT TATCCCTCCA CTTAAATCGA AGGGTTTTTC
CGTATCACCA ATAGGGAGGT GAATTTAGCT TCCCAAAAAG

5690 5700 5710 5720
ACTGCAGATA TGTAGGTTTC CTACGGTGAA TATAAAACTT
TGACGTCTAT ACATCCAAAG GATGCCACTT ATATTTTGAA

5730 5740 5750 5760
ATATCCCGTT GTGTACACGA TAGTACATAG TGTTTATCCC
TATAGGGCAA CACATGTGCT ATCATGTATC ACAAATAGGG

5770 5780 5790 5800
TCCACTTAAA TCGAAGGGTT TTGTGTCAGA CGCGTGTAAG
AGGTGAATTT AGCTTCCCAA AACACAGTCT GCGCACATTC

5810 5820 5830 5840
GAGTGGTCAA CCTTACGACA CATTTAAAAT AATGCGTCCG
CTCACCAGTT GGAATGCTGT GTAAATTTTA TTACGCAGGC

5850 5860 5870 5880
TGGTGCATAC GATAATGCAT AGTGTTTGTC CCTCCACTTA
ACCACGTATG CTATTACGTA TCACAAACAG GGAGGTGAAT

5890 5900 5910 5920
AATCGAAGGG TTGTGTATAT GGATCATGCG GATAAAGTTA
TTAGCTTCCC AACACATATA CCTAGTACGC CTATTTCAAT

5930 5940 5950 5960
TACTGGTGCA GTATAACCCG TTATACACAT AAAATTATGA
ATGACCACGT CATATTGGGC AATATGTGTA TTTTAATACT

5970 5980 5990
GGGATTCGAA TTCCCCCTTA CCTCGGGTAG AGGCCCA 3'
CCCTAAGCTT AAGGGGGAAT GGAGCCCATC TCCGGGT 5'

3. DNA-Sequenz, welche mit einer DNA-Sequenz gemäß Anspruch 1 oder 2 unter den folgenden Bedingungen hybridisieren kann:
(i) wird die DNA-Sequenz auf einem Nitrocellulosefilter fixiert und der Filter bei 37 - 42 °C für eine Zeitdauer von 3 Stunden bis über Nacht in einem Puffer äquilibriert, der 25 mM KPO4 (pH 7,4), 5 x SSC, 5 x Deuhard'sche Lösung [enthaltend 0,2 % Phycol (Molekulargewicht 40000), 0,2 % Polyvinylpyrrolidin (Molekulargewicht: 30000 - 40000) sowie 0,2 % Rinderserumalbumin (Fraktion V)], 50 µg/ml Lachsspermien-DNA und 50 % Formaldehyd enthält,
(ii) wird der Filter anschließend bei 37 - 42 °C für eine Zeitdauer von 6 Stunden bis über Nacht in demselben Puffer äquilibriert, wobei der Puffer dahingehend modifiziert ist, daß er zusätzlich 10 % Dextransulfat und eine DNA-Sequenz gemäß Anspruch 1 oder 2 enthält, und
(iii) wird der Filter danach auf die Hybridisierung zwischen der fixierten DNA und der DNA gemäß Anspruch 1 und 2 getestet.

4. Verfahren zur Herstellung der DNA gemäß einem der Ansprüche 1 - 3, welches die Isolierung eine RNA-Gens eines Odontoglossum Ringspot Virus und die Herstellung einer zu der isolierten, viralen RNA komplementären cDNA umfaßt.

5. Verfahren zur Herstellung der DNA gemäß Anspruch 4, welches außerdem die Klonierung der erhaltenen cDNA umfaßt.

6. Rekombinanter Vektor, der eine gemäß einem der Ansprüche 1 - 3 isolierte DNA umfaßt.

7. Transformand, welcher einen rekombinanten Vektor gemäß Anspruch 6 umfaßt.

8. Transformand gemäß Anspruch 7, bei dem die Wirtszelle aus der aus *E. Coli, Bacillus*, Hefe, *Agrobacterium* und Pflanzenzellen bestehenden Gruppe ausgewählt ist.

9. Verfahren zur Herstellung eines Transformanden, welches die Transformation einer Wirtszelle mit einem rekombinanten Vektor gemäß Anspruch 6 umfaßt.

10. Verfahren zum Nachweis eines mit dem Odontoglossum Ringspot Virus verwandten Genes, welches die Hybridisierung einer Nukleinsäure, die aus der aus RNA-Genen des Odontoglossum Ringspot Virus und der durch reverser Transkription des Virus-RNA-Genes oder Klonierung erhaltenen cDNA bestehenden Gruppe ausgewählt ist, mit einer Probe, bestehend aus der isolierten DNA gemäß einem der Ansprüche 1 - 3, und den Nachweis des hybridisierten Produktes umfaßt.

11. 33K-Protein oder Hüllprotein, hergestellt durch Expression der rekombinanten DNA des ORSV.

12. Peptidfragment zur Verwendung als Testreagenz zum Nachweis von ORSV-Genen, dadurch gekennzeichnet, daß das Peptidfragment durch Expression einer rekombinanten DNA mit einer Sequenz gemäß Anspruch 1 oder 3 erhalten wird.

## Revendications

1. Séquence isolée d'ADN correspondant à (1) un gène du virus Odontoglossum ringspot (ORSV), ou à un fragment de celui-ci, produit par clivage au moyen d'une ou plusieurs enzymes de restriction choisies parmi EcoRI, AccI, PstI, HindIII, HpaI, MluI, et ApaI, ou (2) une séquence complémentaire de (1).

2. Séquence d'ADN suivant la revendication 1, dans laquelle le gène du virus ORSV a la séquence :

10 20 30 40
5' GACTGTTCTT ACTCCGTTGA TCTTCCGGGA AAAACATACG
3' CTGACAAGAA TGAGGCAACT AGAAGGCCCT TTTTGTATGC

50 60 70 80
CTGTGGCGCT TCATAGTATC TATGATATTC CAGCGGATGA
GACACCGCGA AGTATCATAG ATACTATAAG GTCGCCTACT

90 100 110 120
GTTCGGAGCT GCCTTATTAC GAAAGGATGT TCACATATGT
CAAGCCTCGA CGGAATAATG CTTTCCTACA AGTGTATACA

130 140 150 160
TATGCTGCTT TTCATTTTAG TGAGAATCTG CTTCTTGAGA
ATACGACGAA AAGTAAAATC ACTCTTAGAC GAAGAACTCT

170 180 190 200
CTACGTCTGC TCCGCTCGAT GAGATTGGAG CGACGTTTTA
GATGCAGACG AGGCGAGCTA CTCTAACCTC GCTGCAAAAT

210 220 230 240
CAAGTCAGGG GATAGATTAT CCTTCTTCTT TCAAAATGAG
GTTCAGTCCC CTATCTAATA GGAAGAAGAA AGTTTTACTC

250 260 270 280
AGCACATTGA ATTATGAACA TTCTTATAAA AACGTAATTA
TCGTGTAACT TAATACTTGT AAGAATATTT TTGCATTAAT

290 300 310 320
AATATGTTTG CAAGACTTTT TTCCCAGCTT CAAATAGGTT
TTATACAAAC GTTCTGAAAA AAGGGTCGAA GTTTATCCAA

330 340 350 360
CGTCTACCAT AAGGAATTTA TGTGCACCAG AGTTAACACA
GCAGATGGTA TTCCTTAAAT ACACGTGGTC TCAATTGTGT

370 380 390 400
TGGTTTTGTA AGTTCACCAA GGTAGATACT TATTTCCTTT
ACCAAAACAT TCAAGTGGTT CCATCTATGA ATAAAGGAAA

410 420 430 440
TCCGAGGGGT GTATACTAGG GGAGAAGATT CTGAACAATT
AGGCTCCCCA CATATGATCC CCTCTTCTAA GACTTGTTAA

450 460 470 480
TTACACTGCT ATGGATGAAG CATGGGAGTA TAAGAAAACT
AATGTGACGA TACCTACTTC GTACCCTCAT ATTCTTTTGA

490 500 510 520
TTGGCAATGC TTAATAGCGA AAGGACGATC TTTCGCGATC
AACCGTTACG AATTATCGCT TTCCTGCTAG AAAGCGCTAG

530 540 550 560
GCGCTGCGGT TAACTTTTGG TTTCCTAAAG TTAAGGATAT
CGCGACGCCA ATTGAAAACC AAAGGATTTC AATTCCTATA

570 580 590 600
GGTTATTGTG CCGCTTTTTG ATGGTTCCGT CACTTCAGGA
CCAATAACAC GGCGAAAAAC TACCAAGGCA GTGAAGTCCT

610 620 630 640
AAGATGAAAA GAAGTGAAGT CATGGTTAAC AAGGATTTTG
TTCTACTTTT CTTCACTTCA GTACCAATTG TTCCTAAAAC

650 660 670 680
TTTACACCGT TTTAAATCAC ATCCGTACCT ATCAAGACAA
AAATGTGGCA AAATTTAGTG TAGGCATGGA TAGTTCTGTT

690 700 710 720
AGCCTTGACG TATAAAAACG TTCTTTCCTT TGTCGAGTCA
TCGGAACTGC ATATTTTTGC AAGAAAGGAA ACAGCTCAGT

730 740 750 760
ATTAGGTCTC GTGTGATTAT TAATGGTGTG ACTGCAAGAT
TAATCCAGAG CACACTAATA ATTACCACAC TGACGTTCTA

770 780 790 800
CTGAGTGGGA TGTAGACAAG TCTGTGTTAC AGGCGTTATC
GACTCACCCT ACATCTGTTC AGACACAATG TCCGCAATAG

810 820 830 840
TATGACTTTT CTTTTACAGA CGAAGTTGGC TGAAGCAAAA
ATACTGAAAA GAAAATGTCT GCTTCAACCG ACTTCGTTTT

850 860 870 880
GATCAAGTCG TACTTAAAAA ATTTCAAAAA TTTGACGATA
CTAGTTCAGC ATGAATTTTT TAAAGTTTTT AAACTGCTAT

890 900 910 920
CAGTTACTAA TCTTTTCTGG AAACAGATTA GTGATGCTGT
GTCAATGATT AGAAAAGACC TTTGTCTAAT CACTACGACA

930 940 950 960
AGGCGATCTC TTCCCGTCGA TTAAGGAAAG GCTCATTAGC
TCCGCTAGAG AAGGGCAGCT AATTCCTTTC CGAGTAATCG

970 980 990 1000
GGTGGGTTTG TAAAAGTTGC TGAGCAATCT TTACAAATTA
CCACCCAAAC ATTTTCAACG ACTCGTTAGA AATGTTTAAT

1010 1020 1030 1040
AAACGCCTGA TGAGTATATC ACCTTTGCAG ATAAACTGGT
TTTGCGGACT ACTCATATAG TGGAAACGTC TATTTGACCA

1050 1060 1070 1080
TATGGAATAT AAAGCAACTG AAGAGCTTCA ACATCTAGAC
ATACCTTATA TTTCGTTGAC TTCTCGAAGT TGTAGATCTG

1090 1100 1110 1120
ATTAGTAAGC CTCTTGAGAG AGCCGAGAAA TATTATAATG
TAATCATTCG GAGAACTCTC TCGGCTCTTT ATAATATTAC

1130 1140 1150 1160
CTTTATCGGA ACTATCGGTT CTCAAGGAAT GTGACGAGTT
GAAATAGCCT TGATAGCCAA GAGTTCCTTA CACTGCTCAA

1170 1180 1190 1200
TGATATAACG CAATTCAAGA ATTTATGTGA AGAAAAGGAC
ACTATATTGC GTTAAGTTCT TAAATACACT TCTTTTCCTG

1210 1220 1230 1240
ATTGATCCAG ACGTCGTGGC GAAAGTGATC GTCGCCATCA
TAACTAGGTC TGCAGCACCG CTTTCACTAG CAGCGGTAGT

1250 1260 1270 1280
TGAAGAACGA ACTCACACTG CCTTTTAAAA ATCCAACGCC
ACTTCTTGCT TGAGTGTGAC GGAAAATTTT TAGGTTGCGG

1290 1300 1310 1320
TGAAGCATTA TCGGATGCTC TTTCACCGTT ACCCAAAGAC
ACTTCGTAAT AGCCTACGAG AAAGTGGCAA TGGGTTTCTG

1330 1340 1350 1360
TTGGATATGA GATTTGATCT GCTTAAATTA AGTACGTGTG
AACCTATACT CTAAACTAGA CGAATTTAAT TCATGCACAC

1370 1380 1390 1400
CACCCTTTCC AAGCGTTAAA ACTTTAGATA GTGGTTTATT
GTGGGAAAGG TTCGCAATTT TGAAATCTAT CACCAAATAA

1410 1420 1430 1440
ACCAAAGCAA AGCTACGGTG ACGAAAGACA ATTTGAGAGT
TGGTTTCGTT TCGATGCCAC TGCTTTCTGT TAAACTCTCA

1450 1460 1470 1480
CAATCTGTCG TGTCTGTATC TGATTTTCAT CTTAAGAGCG
GTTAGACAGC ACAGACATAG ACTAAAAGTA GAATTCTCGC

1490 1500 1510 1520
TCGAGTCAGT AAAAATGAAA AGTATGTCGT CAGCGGTCTA
AGCTCAGTCA TTTTTACTTT TCATACAGCA GTCGCCAGAT

1530 1540 1550 1560
CACAGGTCCC CTCAAAGTTC AACAGATGAA GAACTATATG
GTGTCCAGGG GAGTTTCAAG TTGTCTACTT CTTGATATAC

1570 1580 1590 1600
GATTACCTGT CCGCATCGAT TTCGGCTACT GTTTCAAATT
CTAATGGACA GGCGTAGCTA AAGCCGATGA CAAAGTTTAA

1610 1620 1630 1640
TATGCAAGGT GCTAAAGGAT GTCTATGGTG CCGACCCAGA
ATACGTTCCA CGATTTCCTA CAGATACCAC GGCTGGGTCT

1650 1660 1670 1680
GTCTGCAGAA AAGTCTGGAG TCTACGATGT AGTGAAAGGC
CAGACGTCTT TTCAGACCTC AGATGCTACA TCACTTTCCG

1690 1700 1710 1720
AAATGGTTAT TAAAACCAAA GGGTAAATGT CACGCTTGGG
TTTACCAATA ATTTTGGTTT CCCATTTACA GTGCGAACCC

1730 1740 1750 1760
GTGTTGCCGA ACTTAACAAT GGCGAGAAAG TCATCGTATT
CACAACGGCT TGAATTGTTA CCGCTCTTTC AGTAGCATAA

1770 1780 1790 1800
ACTCGAATGG GCTGATGGAT TCCCTATTTG TGGTGATTGG
TGAGCTTACC CGACTACCTA AGGGATAAAC ACCACTAACC

1810 1820 1830 1840
CGACGAGTGG CCGTCTCTTC AGATTCCCTT ATTTATTCAG
GCTGCTCACC GGCAGAGAAG TCTAAGGGAA TAAATAAGTC

1850 1860 1870 1880
ATATGGGAAA ACTTCAGACG TTGTTAAGCT GCTTAAAGGA
TATACCCTTT TGAAGTCTGC AACAATTCGA CGAATTTCCT

1890 1900 1910 1920
CGGTGAGCCT GTACCTTCGG ATGCGAAAGT CACGTTAGTA
GCCACTCGGA CATGGAAGCC TACGCTTTCA GTGCAATCAT

1930 1940 1950 1960
GATGGCGTTC CTGGTTGTGG AAAAACAAAG GAAATTCTTG
CTACCGCAAG GACCAACACC TTTTTGTTTC CTTTAAGAAC

1970 1980 1990 2000
AAACTGTGAA TTTTGATGAG GACTTAATAC TTGTTCCTGG
TTTGACACTT AAAACTACTC CTGAATTATG AACAAGGACC

2010 2020 2030 2040
TAAAGAAGCT TGTAAAATGA TTATCAAGAG AGCTAATAAG
ATTTCTTCGA ACATTTTACT AATAGTTCTC TCGATTATTC

2050 2060 2070 2080
TCTGGACATG TAAGAGCGAC AAAGGATAAT GTAAGAACTG
AGACCTGTAC ATTCTCGCTG TTTCCTATTA CATTCTTGAC

2090 2100 2110 2120
TGGATTCGTT CTTAATGCAT TTGAAGCCTA AGACTTATAA
ACCTAAGCAA GAATTACGTA AACTTCGGAT TCTGAATATT

2130 2140 2150 2160
CAAACTGTTT ATCGATGAGG GTTTGATGTT ACATACTGGT
GTTTGACAAA TAGCTACTCC CAAACTACAA TGTATGACCA

2170 2180 2190 2200
TGTGTTAACT TTCTGATAGC ATTGTCCCAT TGTCGTGAAG
ACACAATTGA AAGACTATCG TAACAGGGTA ACAGCACTTC

2210 2220 2230 2240
CAATGGTTTT TGGTGATACT GAACAAATTC CCTTTATAAA
GTTACCAAAA ACCACTATGA CTTGTTTAAG GGAAATATTT

2250 2260 2270 2280
CAGAGTGGCA AATTTCCCTT ATCCGAAACA TTTTGCCACA
GTCTCACCGT TTAAAGGGAA TAGGCTTTGT AAAACGGTGT

2290 2300 2310 2320
CTTGTTTATG ATCATAGGGA GGTTAGGAGG TTATCACTTA
GAACAAATAC TAGTATCCCT CCAATCCTCC AATAGTGAAT

2330 2340 2350 2360
GATGCCCGGC AGATGTTACG CATTTTATGA ACTCCAAATA
CTACGGGCCG TCTACAATGC GTAAAATACT TGAGGTTTAT

2370 2380 2390 2400
TGACGGGAAA GTTTTATGTA CGAATGATGT GATTCGCTCA
ACTGCCCTTT CAAAATACAT GCTTACTACA CTAAGCGAGT

2410 2420 2430 2440
GTTGATGCTG AAGTGGTTAG AGGGAAAGGG GTTTTTAACC
CAACTACGAC TTCACCAATC TCCCTTTCCC CAAAAATTGG

2450 2460 2470 2480
CGAAAAGCAA ACCGCTTAAA GGGAAAATTA TCACCTTCAC
GCTTTTCGTT TGGCGAATTT CCCTTTTAAT AGTGGAAGTG

2490 2500 2510 2520
TCAAAGTGAC AAAGCTGAAC TGAAGGAGCG TGGTTATGAA
AGTTTCACTG TTTCGACTTG ACTTCCTCGC ACCAATACTT

2530 2540 2550 2560
GAAGTTTCAA CTTTTGGTGA AATTAACACC GTACACGAAA
CTTCAAAGTT GAAAACCACT TTAATTGTGG CATGTGCTTT

2570 2580 2590 2600
TCCAAGGTGA GACCTTTGAA GACGTATCGG TTGTTCGTTT
AGGTTCCACT CTGGAAACTT CTGCATAGCC AACAAGCAAA

2610 2620 2630 2640
GACACCAACT CCATTGGAGC TTATATCAAA GAGTTCACCG
CTGTGGTTGA GGTAACCTCG AATATAGTTT CTCAAGTGGC

2650 2660 2670 2680
CACGTTCTTG TTGCATTGAC AAGACATACT AAAAGCTTCA
GTGCAAGAAC AACGTAACTG TTCTGTATGA TTTTCGAAGT

2690 2700 2710 2720
AATATTACTC TGTGGTTCTC GATCCTCTCG TCAAAGTTTG
TTATAATGAG ACACCAAGAG CTAGGAGAGC AGTTTCAAAC

2730 2740 2750 2760
CTCTGATTTA AGCAAGGTGT CAGACTTTAT ACTGGATATG
GAGACTAAAT TCGTTCCACA GTCTGAAATA TGACCTATAC

2770 2780 2790 2800
TATAAGGTCG ATGCCGGGAT CTTATAGCAA TTACAGGTAG
ATATTCCAGC TACGGCCCTA GAATATCGTT AATGTCCATC

2810 2820 2830 2840
GGAGTATCTT CAAAGGCGAA AATCTATTCG TACCATGTCC
CCTCATAGAA GTTTCCGCTT TTAGATAAGC ATGGTACAGG

2850 2860 2870 2880
TAAATCTGGT TATATTTCTG ATATGCAATT TTATTATGAC
ATTTAGACCA ATATAAAGAC TATACGTTAA AATAATACTG

2890 2900 2910 2920
ACTTTGTTAC CTGGAAACAG CACTATTTTA AACGAGTATG
TGAAACAATG GACCTTTGTC GTGATAAAAT TTGCTCATAC

2930 2940 2950 2960
ATGCAGTGAC TATGAATCTT CGTGAAAATA ATCTTAATGT
TACGTCACTG ATACTTAGAA GCACTTTTAT TAGAATTACA

2970 2980 2990 3000
CAAGGATTGC ACGATAGATT TTTCTAAATC TGTTAGTGTC
GTTCCTAACG TGCTATCTAA AAAGATTTAG ACAATCACAG

3010 3020 3030 3040
CCGAGACAAC AACAAGAGTT TTTCACACCA GTTATTCGAA
GGCTCTGTTG TTGTTCTCAA AAAGTGTGGT CAATAAGCTT

3050 3060 3070 3080
CTGCTGCTGA ACGTCCACGT AGTCGTGGTC TCCTTGAAAA
GACGACGACT TGCAGGTGCA TCAGCACCAG AGGAACTTTT

3090 3100 3110 3120
CCTTGTGGCA ATGATAAAAA GAAATTTTAA CTCTCCAGAT
GGAACACCGT TACTATTTTT CTTTAAAATT GAGAGGTCTA

3130 3140 3150 3160
TTAACGGGTA TATTAGATAT TGAAGATACT GCCGAACTTG
AATTGCCCAT ATAATCTATA ACTTCTATGA CGGCTTGAAC

3170 3180 3190 3200
TAGTTAATAA GTTTTGGGAT GCTTACATTA TTGACGAACT
ATCAATTATT CAAAACCCTA CGAATGTAAT AACTGCTTGA

3210 3220 3230 3240
TTCTGGTGGA AATGTTACAC CGATGACTTC AGATGCCTTT
AAGACCACCT TTACAATGTG GCTACTGAAG TCTACGGAAA

3250 3260 3270 3280
CACAGGTGGA TGGCTAAGCA GGAGAAAAGT ACTATTGGTC
GTGTCCACCT ACCGATTCGT CCTCTTTTCA TGATAACCAG

3290 3300 3310 3320
AATTAGCTGA TTTTGACTTT GTTGATTTAC CCGCTATTGA
TTAATCGACT AAAACTGAAA CAACTAAATG GGCGATAACT

3330 3340 3350 3360
TCAATACAAA CATATGATTA AGGCGCAACC TAAACAGAAG
AGTTATGTTT GTATACTAAT TCCGCGTTGG ATTTGTCTTC

3370 3380 3390 3400
CTTGATCTCA GTCCTCAAGA TGAGTATGCT GCACTTCAGA
GAACTAGAGT CAGGAGTTCT ACTCATACGA CGTGAAGTCT

3410 3420 3430 3440
CTATTGTTTA TCACTCAAAG CAGATAAATG CAATTTTCGG
GATAACAAAT AGTGAGTTTC GTCTATTTAC GTTAAAAGCC

3450 3460 3470 3480
TCCTCTTTTT TCCGAATTAA CTAGACAGCT TTTAGAGAGG
AGGAGAAAAA AGGCTTAATT GATCTGTCGA AAATCTCTCC

3490 3500 3510 3520
ATAGACAGTA GCAAATTTTT GTTTTACACC CGGAAAACGC
TATCTGTCAT CGTTTAAAAA CAAAATGTGG GCCTTTTGCG

3530 3540 3550 3560
CCGAACAGAT TGAAGAGTTT TTCTCAGATT TGGATTCGAC
GGCTTGTCTA ACTTCTCAAA AAGAGTCTAA ACCTAAGCTG

3570 3580 3590 3600
GGTGCCAATG GAAGTTCTAG AGTTAGACAT TTCAAAGTAT
CCACGGTTAC CTTCAAGATC TCAATCTGTA AAGTTTCATA

3610 3620 3630 3640
GACAAATCCC AGAACGAATT CCATTGTGCT GTTGAGTACT
CTGTTTAGGG TCTTGCTTAA GGTAACACGA CAACTCATGA

3650 3660 3670 3680
TGATTTGGGA GAAACTTGGT CTTAATGGCT TTCTCGAAGA
ACTAAACCCT CTTTGAACCA GAATTACCGA AAGAGCTTCT

3690 3700 3710 3720
AGTTTGGAAA CAGGGACATA GGAAGACATC CTTGAAGGAT
TCAAACCTTT GTCCCTGTAT CCTTCTGTAG GAACTTCCTA

3730 3740 3750 3760
TACACCGCAG GAATAAAGAC TTGTTTATGG TATCAAAGGA
ATGTGGCGTC CTTATTTCTG AACAAATACC ATAGTTTCCT

3770 3780 3790 3800
AAAGCGGAGA TGTAACTACA TTTATTGGCA ATACAGTTAT
TTTCGCCTCT ACATTGATGT AAATAACCGT TATGTCAATA

3810 3820 3830 3840
TATAGCTGCT TGTCTTGCGT CGATGATACC TATGGATAAG
ATATCGACGA ACAGAACGCA GCTACTATGG ATACCTATTC

3850 3860 3870 3880
GTAATTAAAG CTGCGTTTTG TGGAGATGAT AGTATGCTAT
CATTAATTTC GACGCAAAAC ACCTCTACTA TCATACGATA

3890 3900 3910 3920
ACATTCCAAA GGGACTTGAT CTTCCTGATA TCCAATCTGG
TGTAAGGTTT CCCTGAACTA GAAGGACTAT AGGTTAGACC

3930 3940 3950 3960
AGCGAATTTG ATGTGGAACT TCGAGGCAAA ATTATATCGT
TCGCTTAAAC TACACCTTGA AGCTCCGTTT TAATATAGCA

3970 3980 3990 4000
AAGCGTTACG GATATTTTTG CGGGAGGTAT ATTATTCATC
TTCGCAATGC CTATAAAAAC GCCCTCCATA TAATAAGTAG

4010 4020 4030 4040
ATGACAGAGG TGCCATTGTG TATTACGACC CTCTTAAATT
TACTGTCTCC ACGGTAACAC ATAATGCTGG GAGAATTTAA

4050 4060 4070 4080
AATTTCTAAG TTAGGTTGTA AACACATCAA ATCTTTGGAT
TTAAAGATTC AATCCAACAT TTGTGTAGTT TAGAAACCTA

4090 4100 4110 4120
CATTTGGAAG AATTTAGAAT CTCGCTGTGT GACGTAAGCG
GTAAACCTTA TTAAATCTTA GAGCGACACA CTGCATTCGC

4130 4140 4150 4160
CTTCTTTAAA TAATTGTGCG TATTTTGGTC AATTGAATGA
GAAGAAATTT ATTAACACGC ATAAAACCAG TTAACTTACT

4170 4180 4190 4200
TGCAATTGCT GAGGTCCACA AGACCGCAGT AAATGGGTCG
ACGTTAACGA CTCCAGGTGT TCTGGCGTCA TTTACCCAGC

4210 4220 4230 4240
TTTGCGTTTT GTAGTATTGT TAAGTATCTT TCCGATAAAA
AAACGCAAAA CATCATAACA ATTCATAGAA AGGCTATTTT

4250* 4260 4270 4280
ACCTTTTCAG AACCTTGTTC TACAATGGCT CTAGTACTAA
TGGAAAAGTC TTGGAACAAG ATGTTACCGA GATCATGATT

4290 4300 4310 4320
GGGATAGCAT CAAAATCTCT GAATTTATTA ATTTATCAGC
CCCTATCGTA GTTTTAGAGA CTTAAATAAT TAAATAGTCG

4330 4340 4350 4360
CTCTGAAAAA CTACTACCGT CAGCTCTAAC GGCTGTCAAG
GAGACTTTTT GATGATGGCA GTCGAGATTG CCGACAGTTC

4370 4380 4390 4400
TCGGTACGTA TTTCAAAGGT TGATAAAATT ATAAGTTACG
AGCCATGCAT AAAGTTTCCA ACTATTTTAA TATTCAATGC

4410 4420 4430 4440
AGAATGATAC TTTGTCTGAT ATTGATTTGC TTAAAGGTGT
TCTTACTATG AAACAGACTA TAACTAAACG AATTTCCACA

4450 4460 4470 4480
GAAGTTAGTA GAGAATGGGT ATGTGTGCCT TGCCGGCCTC
CTTCAATCAT CTCTTACCCA TACACACGGA ACGGCCGGAG

4490 4500 4510 4520
GTCGTCACTG GTGAATGGAA TTTACCGGAC AATTGCAAAG
CAGCAGTGAC CACTTACCTT AAATGGCCTG TTAACGTTTC

4530 4540 4550 4560
GTGGTGTGAG TATCTGTCTT GTAGACAAAC GCATGAAAAG
CACCACACTC ATAGACAGAA CATCTGTTTG CGTACTTTTC

4570 4580 4590 4600
AGCTAATGAA GCAACACTTG GCTCTTATCA TACATCTGCT
TCGATTACTT CGTTGTGAAC CGAGAATAGT ATGTAGACGA

4610 4620 4630 4640
TGCAAGAAGA GATTCACCTT TAAAATTATC CCGAATTACA
ACGTTCTTCT CTAAGTGGAA ATTTTAATAG GGCTTAATGT

4650 4660 4670 4680
GTGTCACCAC TGCCGACGCT CTTAAAGGAA TATGGCAAGT
CACAGTGGTG ACGGCTGCGA GAATTTCCTT ATACCGTTCA

4690          4700          4710          4720
TATGACAAAT ATTAGAGGTG TGGAAATGGA AAAGGGTTTT
ATACTGTTTA TAATCTCCAC ACCTTTACCT TTTCCCAAAA

4730          4740          4750          4760
TGCCCATTAT CTTTGGAGTT TGTTTCTATT TGCGTGGTGT
ACGGGTAATA GAAACCTCAA ACAAAGATAA ACGCACCACA

4770          4780          4790          4800
ATTTAAATAA TATAAAACTC GGACTGAGAG AGAAGATTTT
TAAATTTATT ATATTTTGAG CCTGACTCTC TCTTCTAAAA

4810          4820          4830          4840
GAACGTAACA GAAGGAGGGC CCACCGAACT TACTGAAGCA
CTTGCATTGT CTTCCTCCCG GGTGGCTTGA ATGACTTCGT

4850          4860          4870          4880
GTTGTTGATG AGTTCGTGGA GAAAGTTCCT ATGGCTGCTA
CAACAACTAC TCAAGCACCT CTTTCAAGGA TACCGACGAT

4890          4900          4910          4920
GGCTCAAGTC TTTTCGCTCT GTCAACAAAA AGAAACCTAG
CCGAGTTCAG AAAAGCGAGA CAGTTGTTTT TCTTTGGATC

4930          4940          4950          4960
TAACAGTTCT AAGTTTGTTA ATGGAAAATC TAGACTTAAT
ATTGTCAAGA TTCAAACAAT TACCTTTTAG ATCTGAATTA

4970          4980          4990          5000
AGTCGTAATA AATTAAATTA TGAAAATGGT GATAGTGATG
TCAGCATTAT TTAATTTAAT ACTTTTACCA CTATCACTAC

5010          5020          5030          5040
TTGGTATTAG TGTTGTAGAT GATATTGTGG TTGGTAATGG
AACCATAATC ACAACATCTA CTATAACACC AACCATTACC

5050          5060          5070          5080
TGTTAGTGAT ATTCGTATTG ATGATGATTG TGAGTCATTT
ACAATCACTA TAAGCATAAC TACTACTAAC ACTCAGTAAA

5090          5100          5110          5120
GACGCACAAT CTGATTCGTA TTGAATATGT CTTACACTAT
CTGCGTGTTA GACTAAGCAT AACTTATACA GAATGTGATA

5130          5140          5150          5160
TACAGACCCG TCTAAGCTGG CTTATTTAAG CTCGGCTTGG
ATGTCTGGGC AGATTCGACC GAATAAATTC GAGCCGAACC

5170 5180 5190 5200
GCTGACCCCA ATTCACTGAT CAACCTTTGT ACCAATTCTC
CGACTGGGGT TAAGTGACTA GTTGGAAACA TGGTTAAGAG

5210 5220 5230 5240
TGGGTAATCA GTTCCAAACA CAACAAGCTC GAACAACTGT
ACCCATTAGT CAAGGTTTGT GTTGTTCGAG CTTGTTGACA

5250 5260 5270 5280
TCAACAGCAG TTTGCTGATG TTTGGCAGCC GGTTCCTACT
AGTTGTCGTC AAACGACTAC AAACCGTCGG CCAAGGATGA

5290 5300 5310 5320
TTGACCAGTA GGTTCCCTGC AGGCGCTGGT TACTTCAGAG
AACTGGTCAT CCAAGGGACG TCCGCGACCA ATGAAGTCTC

5330 5340 5350 5360
TTTATCGCTA TGATCCTATA TTAGATCCTT TAATAACTTT
AAATAGCGAT ACTAGGATAT AATCTAGGAA ATTATTGAAA

5370 5380 5390 5400
CTTAATGGGT ACTTTTGATA CTCGTAATAG AATAATCGAG
GAATTACCCA TGAAAACTAT GAGCATTATC TTATTAGCTC

5410 5420 5430 5440
GTAGAAAATC CGCAGAATCC GACAACTACG GAAACATTAG
CATCTTTTAG GCGTCTTAGG CTGTTGATGC CTTTGTAATC

5450 5460 5470 5480
ATGCAACTCG TAGAGTTGAT GATGCAACTG TAGCAATAAG
TACGTTGAGC ATCTCAACTA CTACGTTGAC ATCGTTATTC

5490 5500 5510 5520
ATCTGCAATA AATAATCTAT TAAATGAGTT AGTTAGGGGA
TAGACGTTAT TTATTAGATA ATTTACTCAA TCAATCCCCT

5530 5540 5550 5560
ACTGGTATGT ACAATCAAGT CTCATTTGAG ACGATATCTG
TGACCATACA TGTTAGTTCA GAGTAAACTC TGCTATAGAC

5570 5580 5590 5600
GACTTACTTG GACCTCTTCC TAATCATATT TAGGAAAATA
CTGAATGAAC CTGGAGAAGG ATTAGTATAA ATCCTTTTAT

5610 5620 5630 5640
ACGTTGATAG TGTTGAACTA TCCGTGGTGC ATACGATAAT
TGCAACTATC ACAACTTGAT AGGCACCACG TATGCTATTA

5650 5660 5670 5680
GCATAGTGGT TATCCCTCCA CTTAAATCGA AGGGTTTTTC
CGTATCACCA ATAGGGAGGT GAATTTAGCT TCCCAAAAAG

5690 5700 5710 5720
ACTGCAGATA TGTAGGTTTC CTACGGTGAA TATAAAACTT
TGACGTCTAT ACATCCAAAG GATGCCACTT ATATTTTGAA

5730 5740 5750 5760
ATATCCCGTT GTGTACACGA TAGTACATAG TGTTTATCCC
TATAGGGCAA CACATGTGCT ATCATGTATC ACAAATAGGG

5770 5780 5790 5800
TCCACTTAAA TCGAAGGGTT TTGTGTCAGA CGCGTGTAAG
AGGTGAATTT AGCTTCCCAA AACACAGTCT GCGCACATTC

5810 5820 5830 5840
GAGTGGTCAA CCTTACGACA CATTTAAAAT AATGCGTCCG
CTCACCAGTT GGAATGCTGT GTAAATTTTA TTACGCAGGC

5850 5860 5870 5880
TGGTGCATAC GATAATGCAT AGTGTTTGTC CCTCCACTTA
ACCACGTATG CTATTACGTA TCACAAACAG GGAGGTGAAT

5890 5900 5910 5920
AATCGAAGGG TTGTGTATAT GGATCATGCG GATAAAGTTA
TTAGCTTCCC AACACATATA CCTAGTACGC CTATTTCAAT

5930 5940 5950 5960
TACTGGTGCA GTATAACCCG TTATACACAT AAAATTATGA
ATGACCACGT CATATTGGGC AATATGTGTA TTTTAATACT

5970 5980 5990
GGGATTCGAA TTCCCCCTTA CCTCGGGTAG AGGCCCA 3'
CCCTAAGCTT AAGGGGGAAT GGAGCCCATC TCCGGGT 5'

3. Séquence d'ADN capable de s'hybridiser avec une séquence d'ADN suivant la revendication 1, ou la revendication 2, dans les conditions suivantes :
(i) Une séquence d'ADN est fixée sur un filtre en nitrocellulose, et le filtre est équilibré dans un tampon contenant 25 mM de K₃PO₄ (pH 7,4), 5 x SSC, 5 x solution de Deuhardt (contenant 0,2 % de phycol (masse moléculaire : 40 000), 0,2 % de polyvinylpyrrolidone (masse moléculaire : 30 000 - 40 000), et 0,2 % d'albumine de sérum bovin (fraction v), 50 µg/ml d'ADN de sperme de saumon, et 50 % de formaldéhyde, à 37-42°C pendant 3 heures à une nuit.
(ii) le filtre est ensuite équilibré dans le même tampon modifié par un autre contenant 10 % de sulfate de dextran et une séquence d'ADN suivant la revendication 1, ou la revendication 2, à 37-42°C pendant 6 heures à une nuit; et
(iii) le filtre est ensuite testé pour hybridation entre l'ADN fixé et l'ADN suivant la revendication 1 et la revendication 2.

4. Méthode de production d'ADN suivant l'une quelconque des revendications 1 à 3, qui comprend l'isolation du gène de l'ARN du virus Odontoglossum ringspot et la production de l'ADNc complémentaire dudit ARN viral isolé.

5. Méthode de production d'ADN suivant la revendication 4, qui comprend encore un clonage de l'ADNc obtenu.

6. Vecteur recombinant qui comprend l'ADN isolé suivant-l'une quelconque des revendications 1 à 3.

7. Transformant qui comprend un vecteur recombinant suivant la revendication 6.

8. Transformant suivant la revendication 7, dans lequel la cellule hôte est choisie dans le groupe comprenant le bacille E. coli, la levure, l'Agrobacterium, et les cellules végétales.

9. Méthode de production d'un transformant qui comprend la transformation d'une cellule hôte avec un vecteur recombinant suivant la revendication 6.

10. Méthode de contrôle du gène apparenté au virus Odontoglossum ringspot, qui comprend l'hybridation d'un acide nucléique sélectionné à partir du groupe comprenant les gènes d'ARN du virus Odontoglossum ringspot et de l'ADNc obtenu par transcription inverse du gène d'ARN dudit virus ou par clonage, avec une sonde consistant en ADN isolé suivant l'une quelconque des revendications 1 à 3, et la détection d'un produit hybride.

11. Protéine 33K ou protéine COAT produite par expression d'ADN recombinant du virus Odontoglossum ringspot.

12. Fragment peptidique pour l'utilisation comme réactif pour le screening des gènes du virus Odontoglossum ringspot, caractérisé en ce que le fragment peptidique est obtenu par expression d'ADN recombinant comportant une séquence suivant la revendication 1, ou la revendication 3.
